# EUROPEAN PATENT APPLICATION

(11) **EP 4 201 463 A1**
(43) Date of publication of application: **28.06.2023**
(21) Application number: 22212668.2
(22) Date of filing: 09.12.2022
(51) Int. Cl.: A61M 25/00, A61M 25/01, A61M 27/00

(54) **PLEURAL DRAINAGE CATHETER**

(30) Priority: 13.12.2021 US 202163288745 P; 28.03.2022 US 202263324474 P
(71) Applicant: Cook Medical Technologies LLC, Bloomington, IN 47404 (US)
(72) Inventor: DOWELL, Angela R., Lafayette (US); TANNER, Shaun A, West Lafayette (US); FERRARI, Eugene M, Crown Point (US); PUCKETT, Daniel, Dallas (US); ISCH, Andrew P., Lafayette (US); DREWES, David A., Bloomington (US); HARDERT, Kathryn, Bloomington (US); MCGOFF, Adam, Indianapolis (US)
(74) Representative: Leach, Sean Adam

(57) **Abstract**

Drainage catheters (100, 200, 300, 400, 500,600, 700, 800, 900, 1000, 1100, 1200) configured to be visible by echogenicity and/or echopacity are provided. Drainage catheters including layers or marker bands configured to be visible by echogenicity and/or echopacity are further provided.

## Description

### FIELD

The present disclosure relates to medical devices. More particularly, the disclosure relates to polymeric drainage catheters that have good visibility in ultrasound imaging.

### BACKGROUND

Centesis and drainage catheters have remained relatively unchanged over several years, despite great advancements made to procedures and procedural guidelines with respect to ultrasound imaging. Modern medical guidelines advise that healthcare professionals use ultrasound for identification of thoracic anatomy, diagnosis, and procedures, and medical education in the United States trains medical students to be proficient with ultrasound. Ultrasound technology has improved with respect to quality, cost, and accessibility, which has driven increased use of ultrasound in various settings of care, such as reproductive health, gastroenterology, and interventional radiology drainage, and including real-time monitoring of pleural drain placement.

Polymeric devices, such as pleural drainage catheters, are inherently difficult to detect with ultrasound imaging. Due to several general natural challenges associated with ultrasound, including ultrasound being dynamic, the potential to misinterpret images, and the potential for a medical device to disappear by slipping out of plane, it is important to users to have as much of a catheter visible as possible. Minimally invasive techniques to gain access to the pleural cavity, such as Seldinger, are considered blind, because there is no convenient way to perceive the current generation of catheters while using state-of-the-art imaging techniques. Facilitating the use of ultrasound as a regular means of real-time procedural imaging for pleural drainage catheter placement is socially responsible by fulfilling an unmet need that may increase the safety of these procedures. There is also a preference for "brighter" over "dimmer" options, but there is a disadvantage to infinitely increasing brightness of a catheter because the ultrasound may incorrectly interpret the catheter as larger than the catheter actually is. Consequently, there is an ideal range of echogenicity for a polymeric catheter.

For pleural drainage, the distal tip of a catheter may cause harm if the distal tip is inadvertently advanced into critical organs. It is also critical for the function of the catheter and the well-being of the patient that the most proximal opening of the catheter fully resides within the pleural cavity. It is therefore important that the user accurately perceives under ultrasound the locations of the most proximal opening and the distal tip of the catheter.

The pleural cavity, also referred to as the "pleural space," or "interpleural space," is the potential space between the pleurae of the pleural sac that surrounds each lung. A small amount of serous pleural fluid is maintained in the pleural cavity to enable lubrication between the membranes, and also to create a pressure gradient. Most fluid is produced by the exudation in parietal circulation via bulk flow and reabsorbed by the lymphatic system. Thus, pleural fluid is produced and reabsorbed continuously. The composition and volume is regulated by mesothelial cells in the pleura. Typically, only a few milliliters of pleural fluid are present within the intrapleural space. Larger quantities of fluid may accumulate in the pleural space only when the rate of production exceeds the rate of reabsorption. Normally, the rate of reabsorption increases as a physiological response to accumulating fluid, with the reabsorption rate increasing up to 40 times the normal rate before significant amounts of fluid accumulate within the pleural cavity. Thus, a profound increase in the production of pleural fluid-or some blocking of the reabsorbing lymphatic system-is required for fluid to accumulate in the pleural cavity. A pathologic collection of pleural fluid is referred to as a "pleural effusion." Once a pleural effusion is diagnosed, the cause of the effusion must be determined by draining pleural fluid from the pleural cavity.

The accumulation of air or fluid in the pleural space can prevent the lungs from expanding fully during breathing. In some cases, the accumulation may be life-threatening. Current British Thoracic Society guidelines recommend that initial treatment of pleural disease typically involves the placement of a drainage catheter in order to drain the air and/or excess fluid. Ultrasound is routinely used prior to drainage catheter placement to diagnose and characterize pleural effusions. Gaining access to the pleural cavity involves risk, such as over-insertion, which can involve the puncture of organs and blood vessels.

If a drainage catheter could be developed that is echogenic and visible while using ultrasound, it would be a useful contribution to the art. If a drainage catheter could be developed that encouraged the use of ultrasound during catheter placement, it would be an additional useful contribution to the art.

### SUM MARY

In an example, the present disclosure provides a drainage catheter. The drainage catheter includes a tubular body extending along a longitudinal axis. The tubular body includes an outer surface, a lumen extending longitudinally throughout the tubular body, and a distal portion including a tapered distal tip. The lumen includes a lumen surface. The distal portion has a degree of curvature of greater than 0 degrees. The distal portion includes a plurality of holes proximal to the distal tip and spaced apart longitudinally, and an accessory hole proximal to the plurality of holes. The tubular body is configured to be visible by echogenicity and/or echopacity, or the lumen surface or the outer surface is covered by a layer configured to be visible by echogenicity and/or echopacity. The distal portion may include a circular loop in a longitudinal segment of the tubular body such that the distal tip at least approximates an outer surface of the tubular body proximal to the distal tip, the circular loop including an inner circumference and an outer circumference, the inner circumference including the plurality of holes, and the outer circumference including the accessory hole. The distal portion may be configured to be visible by echogenicity and/or echopacity, or may be covered by the layer, the layer extending longitudinally from the distal tip to proximal to the accessory hole. The tubular body or the layer may include from about 70 weight % to about 90 weight % of tungsten uniformly distributed in the tubular body or the layer. The tubular body or the layer may include from about 40 weight % to about 60 weight % of bismuth uniformly distributed in the tubular body or the layer. The tubular body or the layer may be configured to provide a visual confirmation to an operator of a depth of the drainage catheter in a pleural cavity of a patient past the accessory hole. The distal portion may have a degree of curvature of greater than 360 degrees.

In another example, the present disclosure provides a drainage catheter. The drainage catheter includes a tubular body extending along a longitudinal axis. The tubular body includes an outer surface, a lumen extending longitudinally throughout the tubular body, and a distal portion including a tapered distal tip. The lumen includes a lumen surface. The distal portion has a degree of curvature of about 0 degrees. The distal portion includes a plurality of holes proximal to the distal tip and spaced apart longitudinally and circumferentially about a circumference of the distal portion. The tubular body is configured to be visible by echogenicity and/or echopacity, or the lumen surface or the outer surface is covered by a layer configured to be visible by echogenicity and/or echopacity. The tubular body or the layer may include from about 70 weight % to about 90 weight % of tungsten uniformly distributed in the tubular body or the layer. The tubular body or the layer may include from about 40 weight % to about 60 weight % of bismuth uniformly distributed in the tubular body or the layer. The distal portion may be configured to be visible by echogenicity and/or echopacity, or may be covered by layer, the layer extending longitudinally from the distal tip to proximal to the plurality of holes. The tubular body or the layer may be configured to provide a visual confirmation to an operator of a depth of the drainage catheter in a pleural cavity of a patient past the plurality of holes.

In yet another example, the present disclosure provides a drainage catheter. The drainage catheter includes a tubular body extending along a longitudinal axis. The tubular body includes three concentric layers of material extending longitudinally, the layers including an exterior layer, a middle layer, and an interior layer. The tubular body further includes a lumen extending longitudinally throughout the tubular body. The tubular body further includes a tapered distal tip. The tubular body further includes a plurality of holes proximal to the distal tip and spaced apart longitudinally. The exterior layer, the middle layer, or the interior layer is a layer configured to be visible by echogenicity and/or echopacity. The layer configured to be visible by echogenicity and/or echopacity may include from about 70 weight % to about 90 weight % of tungsten uniformly distributed in the layer. The layer configured to be visible by echogenicity and/or echopacity may include from about 40 weight % to about 60 weight % of bismuth uniformly distributed in the layer. The layer configured to be visible by echogenicity and/or echopacity may provide a visual confirmation to an operator of a depth of the drainage catheter in a pleural cavity of a patient past the plurality of holes. The tubular body may include a distal portion including the distal tip and the plurality of holes, the distal portion having a degree of curvature of greater than 0 degrees. The distal portion may include an elliptically-shaped hole proximal to the plurality of holes. The tubular body may include a distal portion including the distal tip and the plurality of holes, the distal portion including the three concentric layers of material. The tubular body may include a distal portion including the distal tip and the plurality of holes, the distal portion having a degree of curvature of about 0 degrees, and the plurality of holes spaced apart circumferentially about a circumference of the tubular body.

In yet another example, the present disclosure provides a drainage catheter. The drainage catheter includes a tubular body extending along a longitudinal axis. The tubular body includes an outer surface, a lumen extending longitudinally throughout the tubular body, and a distal portion including a tapered distal tip. The lumen includes a lumen surface. The distal portion has a degree of curvature of greater than 0 degrees. The distal portion includes a plurality of holes proximal to the distal tip and spaced apart longitudinally, and an accessory hole proximal to the plurality of holes. The tubular body is a marker band, or the lumen surface or the outer surface is covered by a marker band. The marker band is configured to be visible by echogenicity and/or echopacity. The distal portion may include a circular loop in a longitudinal segment of the tubular body such that the distal tip at least approximates an outer surface of the tubular body proximal to the distal tip, the circular loop including an inner circumference and an outer circumference, the inner circumference including the plurality of holes, and the outer circumference including the accessory hole. The distal portion may be, or may be covered by, a marker band, the marker band extending longitudinally from the distal tip to proximal to the accessory hole. The marker band may include from about 70 weight % to about 90 weight % of tungsten uniformly distributed in the marker band. The marker band may include from about 40 weight % to about 60 weight % of bismuth uniformly distributed in the marker band. The marker band may be configured to provide a visual confirmation to an operator of a depth of the drainage catheter in a pleural cavity of a patient past the accessory hole. The distal portion may have a degree of curvature of greater than 360 degrees.

In yet another example, the present disclosure provides a drainage catheter. The drainage catheter includes a tubular body extending along a longitudinal axis. The tubular body includes an outer surface, a lumen extending longitudinally throughout the tubular body, and a distal portion including a tapered distal tip. The lumen includes a lumen surface. The distal portion has a degree of curvature of about 0 degrees. The distal portion includes a plurality of holes proximal to the distal tip and spaced apart longitudinally and circumferentially about a circumference of the distal portion. The tubular body is a marker band, or the lumen surface or the outer surface is covered by a marker band. The marker band is configured to be visible by echogenicity and/or echopacity. The marker band may include from about 70 weight % to about 90 weight % of tungsten uniformly distributed in the marker band. The marker band may include from about 40 weight % to about 60 weight % of bismuth uniformly distributed in the marker band. The distal portion may be, or may be covered by, a marker band, the marker bad extending longitudinally from the distal tip to proximal to the plurality of holes. The marker band may be configured to provide a visual confirmation to an operator of a depth of the drainage catheter in a pleural cavity of a patient past the plurality of holes.

In yet another example, the present disclosure provides a drainage catheter. The drainage catheter includes a tubular body extending along a longitudinal axis. The tubular body includes three concentric layers of material extending longitudinally, the layers including an exterior layer, a middle layer, and an interior layer. The tubular body further includes a lumen extending longitudinally throughout the tubular body. The tubular body further includes a tapered distal tip. The tubular body further includes a plurality of holes proximal to the distal tip and spaced apart longitudinally. The exterior layer, the middle layer, or the interior layer is a marker band configured to be visible by echogenicity and/or echopacity. The marker band may include from about 70 weight % to about 90 weight % of tungsten uniformly distributed in the marker band. The marker band may include from about 40 weight % to about 60 weight % of bismuth uniformly distributed in the marker band. The marker band may be configured to provide a visual confirmation to an operator of a depth of the drainage catheter in a pleural cavity of a patient past the plurality of holes. The tubular body may include a distal portion including the distal tip and the plurality of holes, and wherein the distal portion has a degree of curvature of greater than 0 degrees. The distal portion may include an elliptically-shaped hole proximal to the plurality of holes. The tubular body may include a distal portion including the distal tip and the plurality of holes, and wherein the distal portion includes the three concentric layers of material. The tubular body may include a distal portion including the distal tip and the plurality of holes, wherein the distal portion has a degree of curvature of about 0 degrees, and wherein the plurality of holes is spaced apart circumferentially about a circumference of the tubular body.

In yet another example, the present disclosure provides a drainage catheter. The drainage catheter includes a tubular body extending along a longitudinal axis. The tubular body includes a lumen extending longitudinally throughout the tubular body, a distal portion including a tapered distal tip, and a marker band encircling the tubular body proximal to the distal tip. The distal portion has a degree of curvature of greater than 0 degrees. The distal portion includes a plurality of holes spaced apart longitudinally, the plurality of holes distal to the marker band and proximal to the distal tip. The distal portion includes an elliptically-shaped hole distal to the marker band. The marker band and the distal tip are configured to be visible by echogenicity and/or echopacity. The distal portion may include a circular loop in a longitudinal segment of the tubular body such that the distal tip at least approximates an outer surface of the tubular body proximal to the distal tip, the circular loop including an inner circumference and an outer circumference; wherein the inner circumference includes the plurality of holes; and wherein the outer circumference includes the elliptically-shaped hole. The marker band and the distal tip each may include from about 70 weight % to about 90 weight % of tungsten. The marker band and the distal tip each may include from about 40 weight % to about 60 weight % of bismuth. The tubular body may include from about 1 weight % to about 10 weight % of a particulate additive including spheres, microspheres, and/or nanospheres. The particulate additive may include glass microspheres of a particle size of from about 2 microns to about 100 microns. The tubular body may include graduated depth indicator lines spaced apart longitudinally and proximal to the plurality of holes. The marker band may be configured to provide a visual confirmation to an operator of a depth of the drainage catheter in a pleural cavity of a patient past a most proximal hole of the plurality of holes. The marker band may be at least 5 millimeters wide longitudinally.

In yet another example, the present disclosure provides a drainage catheter. The drainage catheter includes a tubular body extending along a longitudinal axis. The tubular body includes a lumen extending longitudinally throughout the tubular body, the lumen having a cross-sectional inner diameter, a distal portion including a tapered distal tip, and a marker band encircling the tubular body proximal to the distal tip. The distal portion has a degree of curvature of 0 degrees. The distal portion includes a plurality of holes spaced apart longitudinally and circumferentially about the circumference of the distal portion, the plurality of holes distal to the marker band and proximal to the distal tip. The marker band and the distal tip are configured to be visible by echogenicity and/or echopacity. The marker band and the distal tip each may include from about 70 weight % to about 90 weight % of tungsten. The marker band and the distal tip each may include from about 40 weight % to about 60 weight % of bismuth. The tubular body may include from about 1 weight % to about 10 weight % of a particulate additive including spheres, microspheres, and/or nanospheres. The particulate additive may include glass microspheres of a particle size of from about 2 microns to about 100 microns. The tubular body may include graduated depth indicator lines spaced apart longitudinally and proximal to the plurality of holes. The marker band may be configured to provide a visual confirmation to an operator of a depth of the drainage catheter in a pleural cavity of a patient past a most proximal hole of the plurality holes.

In yet another example, the present disclosure provides a drainage catheter. The drainage catheter includes a tubular body extending along a longitudinal axis. The tubular body includes a lumen extending longitudinally throughout the tubular body, a distal portion including a tapered distal tip, and a marker band encircling the tubular body proximal to the distal tip. The distal portion has a degree of curvature of at least 360 degrees. The distal portion includes a plurality of holes spaced apart longitudinally, the plurality of holes distal to the marker band and proximal to the distal tip. The distal portion includes an elliptically-shaped hole distal to the marker band. The marker band and the distal tip are configured to be visible by echogenicity and/or echopacity. The distal portion may include a circular loop in a longitudinal segment of the distal portion such that the distal tip at least approximates an outer surface of the tubular body proximal to the distal tip, the circular loop including an inner circumference and an outer circumference. The inner circumference may include the plurality of holes. The outer circumference may include an elliptically-shaped hole distal to the marker band. The marker band and the distal tip each may include from about 70 weight % to about 90 weight % of tungsten. The marker band and the distal tip each may include from about 40 weight % to about 60 weight % of bismuth.

Further areas of applicability will become apparent from the description provided herein. It should be understood that the description and specific examples are intended for purposes of illustration only and are not intended to limit the scope of the present disclosure.

### DRAWINGS

In order that the present disclosure may be well understood, there will now be described various forms thereof, given by way of example, reference being made to the accompanying drawings. The components in the figures are not necessarily to scale. Moreover, in the figures, like-referenced numerals designate corresponding parts throughout the different views.
FIG. **1** illustrates a perspective view of an example of a drainage catheter according to the principles of the present disclosure, having a small-bore catheter size;
FIG. **2** illustrates a perspective view of another example of a drainage catheter according to the principles of the present disclosure, having a mid-bore catheter size;
FIG. **3** illustrates a perspective view of yet another example of a drainage catheter according to the principles of the present disclosure, having a large-bore catheter size;
FIG. **4** illustrates an ultrasound of yet another example of a drainage catheter according to the principles of the present disclosure upon insertion into a phantom pleural cavity filled with fluid;
FIG. **5** illustrates an ultrasound of yet another example of a drainage catheter according to principles of the present disclosure upon insertion into a phantom pleural cavity filled with fluid;
FIG. **6** illustrates an ultrasound of a simulated porcine pleural effusion in which yet another example of a drainage catheter according to the principles of the present disclosure has been identified and labeled;
FIG. **7** illustrates a longitudinal cross-sectional view of a distal portion of yet another example of a drainage catheter according to the principles of the present disclosure, made up of material including or incorporating a particulate additive that is large-particle tungsten;
FIG. **8** illustrates a perspective view of a distal portion of yet another example of a drainage catheter according to the principles of the present disclosure;
FIG. **9** illustrates a perspective view of a distal portion of yet another example of a drainage catheter according to the principles of the present disclosure;
FIG. **10** illustrates a perspective view of yet another example of a drainage catheter, with a distal portion with a degree of curvature of greater than 360 degrees, according to the principles of the present disclosure;
FIG. **11** illustrates a perspective view of yet another example of a drainage catheter, with a distal portion with a degree of curvature of approximately 360 degrees, according to the principles of the present disclosure;
FIG. **12A** illustrates a perspective view of a distal portion of yet another example of a drainage catheter, with a degree of curvature of greater than 270 degrees and less than 360 degrees, according to the principles of the present disclosure;
FIG. **12B** illustrates a side view of the distal portion of the example of a drainage catheter illustrated in FIG. **12A****;**
FIG. **13A** illustrates a perspective view of a distal portion of yet another example of a drainage catheter, with a degree of curvature of approximately 180 degrees, according to the principles of the present disclosure;
FIG. **13B** illustrates a side view of the distal portion of the example of a drainage catheter illustrated in FIG. **13A****;**
FIG. **14A** illustrates a perspective view of a distal portion of yet another example of a drainage catheter, with a degree of curvature of greater than 0 degrees and less than 90 degrees, according to the principles of the present disclosure;
FIG. **14B** illustrates a side view of the distal portion of the example of a drainage catheter illustrated in FIG. **14A****;**
FIG. **15** illustrates a perspective view of yet another example of a drainage catheter according to the principles of the present disclosure;
FIG. **16** illustrates a perspective view of a distal portion of yet another example of a drainage catheter, with a degree of curvature of greater than 360 degrees, according to the principles of the present disclosure;
FIG. **17** illustrates a perspective view of a distal portion of yet another example of a drainage catheter, with a degree of curvature of approximately 360 degrees, according to the principles of the present disclosure;
FIG. **18** illustrates a perspective view of yet another example of a drainage catheter according to the principles of the present disclosure;
FIG. **19** illustrates a perspective view of yet another example of a drainage catheter according to the principles of the present disclosure.
FIG. **20** illustrates an ultrasound of yet another example of a drainage catheter according to the principles of the present disclosure upon insertion into a phantom pleural cavity filled with fluid;
FIG. **21** illustrates an ultrasound of yet another example of a drainage catheter according to the principles of the present disclosure upon insertion into a phantom pleural cavity filled with fluid;
FIG. **22** illustrates an ultrasound of yet another example of a drainage catheter according to the principles of the present disclosure upon insertion into a phantom pleural cavity filled with fluid;
FIG. **23** illustrates an ultrasound of yet another example of a drainage catheter according to the principles of the present disclosure upon insertion into a phantom pleural cavity filled with fluid;
FIG. **24** illustrates an ultrasound of a state-of-the-art pleural drainage catheter upon insertion into a phantom pleural cavity filled with fluid;
FIG. **25** illustrates the examples of drainage catheters according to the principles of the present disclosure of FIGs. **20 - 23** shown under fluoroscopy;
FIG. **26** illustrates the examples of drainage catheters of FIGs. **20** - **22** and **24** shown under fluoroscopy.
FIG. **27A** illustrates a perspective view of a distal portion of yet another example of a drainage catheter, with a degree of curvature of greater than 270 degrees and less than 360 degrees, according to the principles of the present disclosure;
FIG. **27B** illustrates a side view of the distal portion of the example of a drainage catheter illustrated in FIG. **27A****;**
FIG. **28A** illustrates a perspective view of a distal portion of yet another example of a drainage catheter, with a degree of curvature of approximately 180 degrees, according to the principles of the present disclosure;
FIG. **28B** illustrates a side view of the distal portion of the example of a drainage catheter illustrated in FIG. **28A****;**
FIG. **29A** illustrates a perspective view of a distal portion of yet another example of a drainage catheter, with a degree of curvature of greater than 0 degrees and less than 90 degrees, according to the principles of the present disclosure; and
FIG. **29B** illustrates a side view of the distal portion of the example of a drainage catheter illustrated in FIG. **29A****.**

The drawings described herein are for illustration purposes only and are not intended to limit the scope of the present disclosure in any way.

### DETAILED DESCRIPTION

The following description is merely exemplary in nature and is not intended to limit the present disclosure, application, or uses. It should be understood that throughout the drawings, corresponding reference numerals indicate like or corresponding parts and features.

In adding reference denotations to elements of each drawing, although the same elements are displayed on a different drawing, it should be noted that the same elements have the same denotations. In addition, in describing one aspect of the present disclosure, if it is determ ined that a detailed description of related well-known configurations or functions blurs the gist of one aspect of the present disclosure, it will be omitted.

In the following discussion, the terms "proximal" and "distal" will be used to describe the opposing axial ends of the device, as well as the axial ends of various component features. The term "proximal" is used in its conventional sense to refer to the end of the device (or component) that is closest to the medical professional during use of the assembly. The term "distal" is used in its conventional sense to refer to the end of the device (or component) that is initially inserted into the patient, or that is closest to the patient during use. The term "longitudinal" will be used to refer to an axis that aligns with the proximal-distal axis of the device (or component). The terms "radially" and "radial" will be used to refer to elements, surfaces, or assemblies relative to one another that may extend perpendicularly from a longitudinal axis. The terms "circumference," "circumferentially," and "circumferential" may be used to refer to elements, surfaces, or assemblies relative to one another encircling a longitudinal axis at a radius. The terms "inner circumference" and "outer circumference" may be used to refer to elements or surfaces relative to one another in particular longitudinal portions of a device, where a particular longitudinal portion is shaped into a circular loop, such that the circular loop has an inner circumference corresponding to the longitudinal portion along a first side of an outer surface of the device; and an outer circumference that is larger than the inner circumference corresponding to the longitudinal portion along a second side of the outer surface of the device that is opposite the first side.

The uses of the terms "a" and "an" and "the" and similar referents in the context of describing the present disclosure (especially in the context of the following claims) are to be construed to cover both the singular and the plural, unless otherwise indicated herein or clearly contradicted by context. The use of the term "plurality of" is defined by the Applicant in its broadest sense, superseding any other implied definitions or limitations hereinbefore or hereinafter unless expressly asserted by the Applicant to the contrary, to mean a quantity of more than one. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context.

As used herein the terms "comprise(s)," "include(s)," "having," "has," "can," "contain(s)," and variants thereof, are intended to be open-ended transitional phrases, terms, or words that do not preclude the possibility of additional acts or structures. The present description also contemplates other embodiments "comprising," "consisting of," and "consisting essentially of," the examples or elements presented herein, whether explicitly set forth or not.

In describing elements of the present disclosure, the terms 1^{st}, 2^{nd}, first, second, A, B, (a), (b), and the like may be used herein. These terms are only used to distinguish one element from another element, but do not limit the corresponding elements irrespective of the nature or order of the corresponding elements.

Unless otherwise defined, all terms used herein, including technical or scientific terms, have the same meanings as those generally understood by those skilled in the art to which the present disclosure pertains. Such terms as those defined in a generally used dictionary are to be interpreted as having meanings equal to the contextual meanings in the relevant field of art.

As used herein, the term "about," when used in the context of a numerical value or range set forth means a variation of ±15%, or less, of the numerical value. For example, a value differing by ±15%, ±14%, ±10%, or ±5%, among others, would satisfy the definition of "about," unless more narrowly defined in particular instances.

As used herein, the terms "radiopacity" and "radiopaque" refer to the opacity of a material to radiation of the radio wave and X-ray portions of the electromagnetic spectrum, or the relative inability of such radiation to pass through the material.

As used herein, the term "echogenicity" refers to the ability of a material to reflect or bounce an echo or return a signal in ultrasound examination. Echogenicity is higher when a surface bouncing a sound echo reflects increased sound waves. As used herein, the term "echopacity" refers to the opacity of a material to sound waves, or the relative inability of sound waves to pass through the material or return a signal in ultrasound examination, or the disruption of sound waves, which may create an acoustic shadow that may be considered an artifact.

As used herein, the terms "extrusion" refers to a standard technique known to those of ordinary skill in the art, in which a raw material is converted into a product of defined shape and density by forcing it through a die under defined conditions. An extruder is generally composed of two different parts: a conveying system and a die system. The conveying system transports material through a barrel via the action of Archimedes' infinite screws, which may also impart a degree of distributive mixing if needed. The die system then forms the material into the desired shape. Extrudates are generally produced by heating and then softening a composition including a thermoplastic polymer, followed by extrusion of the molten mass through a die, resulting in the production of cylinders or films depending on the shape of the die. Other excipients, such as surfactants, salts, superdisintegrants, plasticizers, UV stabilizers, and antioxidants may be added during the extrusion process if required. The most common additives are plasticizers, which facilitate the extrusion process by reducing the glass transition temperature of the polymers. The quality of the final product may be fine-tuned by modifying the excipients.

As used herein, the terms "ellipse," "elliptical," and "elliptically-shaped" refer to a geometric shape in the form of a closed curve that results from the intersection of a cone or a cylinder by a plane. An ellipse has a major axis and a minor axis, each of about which the ellipse is axially symmetric. An ellipse surrounds two focal points, such that for all points on the ellipse, the sum of the distances to the two focal points is a constant. A circle is an example of an ellipse resulting from an intersection of a cone or a cylinder by a plane that is perpendicular to the height of the cone or the cylinder, and in which the two focal points are the same point.

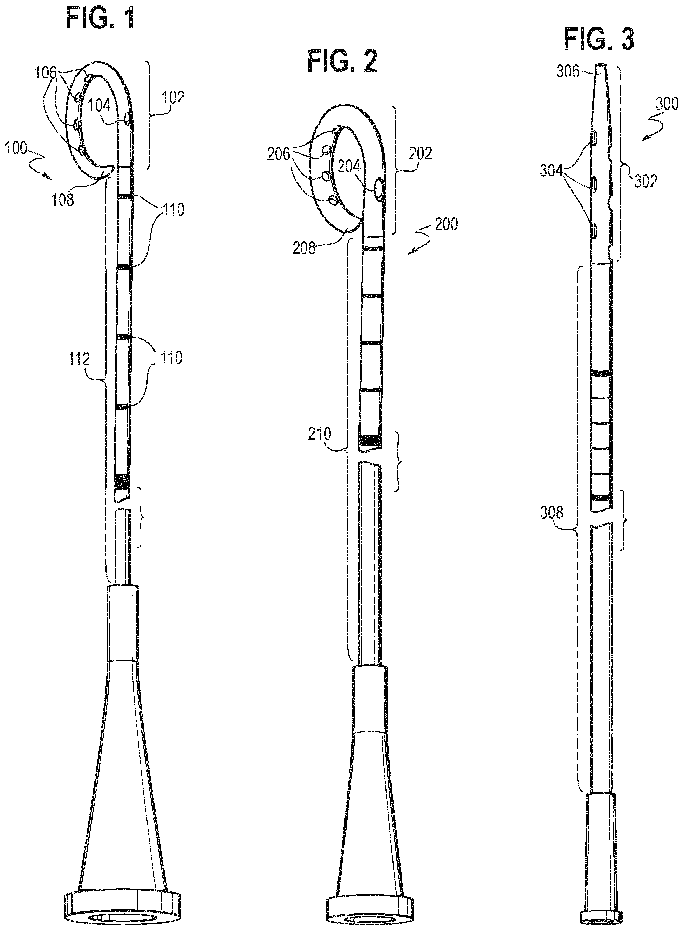

Referring to FIG. **1****,** an example of a drainage catheter **100** is illustrated. Drainage catheter **100** has a small-bore catheter size, for example a 6 French catheter size. Drainage catheter **100** includes a tubular body that extends longitudinally from proximal portion **112** to distal portion **102.** Distal portion **102** includes tapered distal tip **108.** Drainage catheter **100** includes an outer surface and a lumen extending longitudinally throughout drainage catheter **100** from proximal portion **112** to distal tip **108.** Drainage catheter **100** has a longitudinal cross-sectional outer diameter. The lumen extending longitudinally throughout drainage catheter **100** has a longitudinal cross-sectional inner diameter that is shorter than the longitudinal cross-sectional outer diameter of drainage catheter **100.** As illustrated in FIG. **1****,** distal portion **102** may include a degree of curvature in a longitudinal segment of distal portion **102** such that distal tip **108** may at least approximate the outer surface of drainage catheter **100** proximal to distal tip **108.** Distal tip **108** may confront the outer surface of drainage catheter **100** proximal to distal tip **108.** As a consequence of the degree of curvature in the longitudinal segment of distal portion **102,** a first side of the outer surface of drainage catheter **100** corresponds to an inner curvature, and a second side of the outer surface of drainage catheter **100** opposite to the first side corresponds to an outer curvature of the degree of curvature. As illustrated in FIG. **1****,** the first side faces towards proximal portion **112** of drainage catheter **100** and the second side faces away from proximal portion **112** of drainage catheter. The degree of curvature minimizes the linear footprint of drainage catheter **100** in the pleural space. The degree of curvature also anchors drainage catheter **100** in a correct location, thereby reducing the chance of displacement or removal of drainage catheter **100** from the pleural space. The degree of curvature also provides a landmark when imaging under ultrasound and fluoroscopy.

The first side of the outer surface of drainage catheter **100** includes a plurality of holes **106** spaced apart longitudinally about the inner curvature of distal portion **102.** Each of the plurality of holes **106** may have a width corresponding to about a diameter of the lumen extending longitudinally throughout drainage catheter **100.** Positioning plurality of holes **106** on the degree of curvature on the first side of distal portion **102** reduces the probability of one or more of plurality of holes **106** becoming blocked by an organ or tissue. Plurality of holes **106** may include 2 holes, 3 holes, 4 holes, 5 holes, 6 holes, 7 holes, 8 holes, 9 holes, 10 holes, or more than 10 holes longitudinally spaced apart about the degree of curvature of distal portion **102.** Optimizing the number of holes in plurality of holes **106** may maintain the strength of drainage catheter **100** without sacrificing drainage performance.

The second side of distal portion **102** includes accessory hole **104** proximal to the most proximal of plurality of holes **106.** Accessory hole **104** may be configured for insertion of a trocar device. Placement of accessory hole **104** on the outer curvature of the degree of curvature of distal portion **102** makes trocar insertion easier. Accessory hole **104** is also easily identifiable under ultrasound because of the unique shape of elliptically-shaped hole **104.** Accessory hole **104** is an important landmark, because it is the most proximal hole of drainage catheter **100,** and assures the user of drainage catheter **100** that all holes of drainage catheter **100** are in the pleural space. Accessory hole **104** may be used for the "rapid exchange" Seldinger technique, permitting use of a shorter wire guide (as the wire does not have to traverse the length of drainage catheter **100**). Accessory hole **104** also increases hub options because the wire does not have to traverse the hub. Accessory hole **104** must be large enough to accommodate a straightening stiffener, which may have a slightly smaller outer diameter than the inner diameter of drainage catheter **100.** In certain examples, accessory hole **104** may be elliptically-shaped. The elliptical shape may be particularly important, and the major axis of the ellipse should be parallel to the longitudinal axis of drainage catheter **100.** The elliptical shape must have a minor axis larger than the maximum diameter of each of the plurality of holes **106.** In other examples, the second side of distal portion **102** may include an access hole proximal to the most proximal of plurality of holes **106.** In still other examples, the second side of distal portion **102** may include a device hole proximal to the most proximal of plurality of holes **106.**

In proximal portion **112,** graduated depth indicator lines **110** are incrementally spaced apart longitudinally and configured to provide optical perception of insertion depth of drainage catheter **100** into a patient. Graduated depth indicator lines **110** provide precise insertion control and facilitate post-operative maintenance.

Distal portion **102,** from distal tip **108** to proximal to accessory hole **104,** may be, or include, material(s) configured to provide drainage catheter **100** with desirable echopacity and/or echogenicity, for example, in the form of an outer layer of material covering distal portion **102.** The drainage catheters of the present invention exhibit increased echogenicity compared to drainage catheters currently on the market. The drainage catheters of the present invention also have increased radiopacity, because distal portion **102** is distinguishable from proximal portion **112** under fluoroscopy.

Distal portion **102** may include a material having a defined shape and density. Distal portion **102** may be, or include, a material that is an output of a conveying system including distributive mixing and/or a die system. Distal portion **102** may be, in whole or in part, made up of a flexible base thermoplastic material, such as ultrathane, including a particulate additive incorporated into the thermoplastic material. In certain examples, the thermoplastic material including or incorporating a particulate additive may be an outer layer covering a base ultrathane layer making up distal portion **102** and thermoplastic portion, in which case the outer layer includes plurality of holes **106** and accessory hole **104** to match the plurality of holes **106** and accessory hole **104** in the base ultrathane layer. The thermoplastic material may be selected from a group consisting of a polyester elastomer, a polyurethane polymer, a polyamide elastomer, and a polyether block amide. The particulate additive is configured to introduce echopacity and/or echogenicity to drainage catheter **100.** In certain examples, the thermoplastic material may include or incorporate two or more particulate additives. By incorporating particulate additive(s), the thickness of distal portion **102** may modify the mechanical properties of drainage catheter **100** without modifying the base ultrathane material mechanical properties. Further, distal portion **102,** or the component or layer thereof, including or incorporating particulate additive(s), may be configured to be thin enough to retain base ultrathane material mechanical properties while adding echogenicity. By incorporating particulate additive(s) into the base ultrathane material making up all or part of distal portion **102** from distal tip **108** to proximal to elliptically-shaped hole **102,** a user may be able to identify distal tip **108** and plurality of holes **106,** as well as accessory hole **104** when drainage catheter **100** is in the pleural space, for proper placement. Identification of distal tip **108** and accessory hole **104** is important for successful placement of drainage catheter **100.** Identification of distal tip **108** is important to ensure that a user does not over-insert drainage catheter **100** and damage organs. Identification of accessory hole **104** is important so a user knows when all of plurality of holes **106** and accessory hole **104** are located in the plural space and creation of a pneumothorax may be avoided. The material incorporating particulate additive(s) may subsequently be attached to the base thermoplastic material by a physical attachment method such as butt-welding.

In certain examples, a particulate additive may include barium sulfate. In other examples, a particulate additive may include bismuth oxalate. In still other examples, a particulate additive may include bismuth oxychloride. In still other examples, a particulate additive may include bismuth sulfate. In still other examples, a particulate additive may include tungsten in particulate form. In still other examples, a particulate additive may include tungsten carbide. In still other examples, a particulate additive may include aluminum silicate particles. In still other examples, a particulate additive may include calcium carbonate. In still other examples, a particulate additive may include calcium hydroxide, and/or a hydrate thereof. In still other examples, a particulate additive may include calcium oxide or quicklime. In still other examples, a particulate additive may include calcium sulfate. In still other examples, a particulate additive may include clay. In still other examples, a particulate additive may include gypsum. In still other examples, a particulate additive may include mica. In still other examples, a particulate additive may include muscovite mica. In still other examples, a particulate additive may include phlogopite mica. In still other examples, a particulate additive may include micaceous iron oxide. In still other examples, a particulate additive may include talc. In particularly preferred examples, a particulate additive may include a barium species. In other particularly preferred examples, a particulate additive may include a bismuth species. In other particularly preferred examples, a particulate additive may include a tungsten species.

In the above examples of particulate additives, a fill rate of a particulate additive may be consistent at a fixed additive-to-polymer volume ratio but may vary greatly in weight % as a consequence of density of the particulate additive and the thermoplastic material included in drainage catheter **100.** In certain examples, the weight % of the particulate additive may be from about 10 weight %, or from about 15 weight %, or from about 20 weight %, or from about 25 weight %, or from about 30 weight %, or from about 35 weight %, or from about 40 weight %, or from about 45 weight %, or from about 50 weight %, or from about 55 weight %, or from about 60 weight %, or from about 65 weight %, or from about 70 weight %, or from about 75 weight %, or from about 80 weight %, or from about 85 weight % to about 90 weight %; or the particulate additive may be from about 10 weight % to about 15 weight %, or to about 20 weight %, or to about 25 weight %, or to about 30 weight %, or to about 35 weight %, or to about 40 weight %, or to about 45 weight %, or to about 50 weight %, or to about 55 weight %, or to about 60 weight %, or to about 65 weight %, or to about 70 weight %, or to about 75 weight %, or to about 80 weight %, or to about 85 weight %; or any other range of from about one of the above minima to about one of the above maxima. In particularly preferred examples, the weight % of particulate additive may be from about 40 to 60 weight % bismuth, or about 50 weight % bismuth. In other particularly preferred examples, the weight % of particulate additive may be about 15 to about 30 weight % barium, or about 25 weight % barium. In other particularly preferred examples, the weight % of particulate additive may be from about 60 to about 90 weight % tungsten, or up to about 75 weight % tungsten.

In certain examples, a particulate additive may have a particle diameter preferably from about 2 microns to about 200 microns, more preferably from about 20 to about 50 microns, and most preferably from about 10 to about 20 microns, including from about 4 microns, or from about 6 microns, or from about 8 microns, or from about 10 microns, or from about 12 microns, or from about 14 microns, or from about 16 microns, or from about 18 microns, or from about 20 microns, to about 200 microns; or from about 2 microns to about 10 microns, or to about 12 microns, or to about 14 microns, or to about 16 microns, or to about 18 microns, or to about 20 microns, or to about 30 microns, or to about 40 microns, or to about 50 microns, or to about 60 microns, or to about 70 microns, or to about 80 microns, or to about 90 microns, or to about 100 microns, or to about 110 microns, or to about 120 microns, or to about 130 microns, or to about 140 microns, or to about 150 microns, or to about 160 microns, or to about 170 microns, or to about 180 microns, or to about 190 microns; or any other range of from about one of the above minima to about one of the above maxima.

Drainage catheter **100** may be of a catheter size within a range of catheter sizes of from 4 French ("Fr.") to 40 Fr. As will be understood to those of skill in the art, the French scale or French gauge system commonly used to measure the size of a catheter refers to three times the length, measured in millimeters ("mm") of the outer diameter of the catheter. For example, a round catheter of 1 French will have an outer diameter of 1/3 millimeters; a round catheter of 3 French will have an outer diameter of 1 millimeter. In certain examples, a drainage catheter may have a catheter size of from 4 French to 40 French, including from 5 French, or from 6 French, or from 7 French, or from 8 French, or from 9 French, or from 10 French, or from 11 French, or from 12 French, or from 13 French, or from 14 French, or from 15 French, or from 16 French, or from 17 French, or from 18 French, or from 19 French, or from 20 French, or from 21 French, or from 22 French, or from 23 French, or from 24 French, or from 25 French, or from 26 French, or from 27 French, or from 28 French, or from 29 French, or from 30 French, or from 31 French, or from 32 French, or from 33 French, or from 34 French, or from 35 French, or from 36 French, or from 37 French, or from 38 French, or form 39 French to 40 French; or from 4 French to 5 French, or to 6 French, or to 7 French, or to 8 French, or to 9 French, or to 10 French, or to 11 French, or to 12 French, or to 13 French, or to 14 French, or to 15 French, or to 16 French, or to 17 French, or to 18 French, or to 19 French, or to 20 French, or to 21 French, or to 22 French, or to 23 French, or to 24 French, or to 25 French, or to 26 French, or to 27 French, or to 28 French, or to 29 French, or to 30 French, or to 31 French, or to 32 French, or to 33 French, or to 34 French, or to 35 French, or to 36 French, or to 37 French, or to 38 French, or to 39 French, or to 40 French; or any other range of from one of the above minima to one of the above maxima. In an example, a drainage catheter may preferably have a catheter size of from 5 French to 28 French.

In examples of drainage catheters of the present disclosure, the degree of curvature in a distal portion of a drainage catheter may be about 0 degrees, greater than 0 degrees, greater than 0 degrees to less than 360 degrees, approximately 360 degrees, at least 360 degrees, or greater than 360 degrees. In certain examples, the degree of curvature can be from greater than 0 degrees, or from 10 degrees, or from 20 degrees, or from 30 degrees, or from 40 degrees, or from 50 degrees, or from 60 degrees, or from 70 degrees, or from 80 degrees, or from 90 degrees, or from 100 degrees, or from 110 degrees, or from 120 degrees, or from 130 degrees, or from 140 degrees, or from 150 degrees, or from 160 degrees, or from 170 degrees, or from 180 degrees, or from 190 degrees, or from 200 degrees, or from 210 degrees, or from 220 degrees, or from 230 degrees, or from 240 degrees, or from 250 degrees, or from 260 degrees, or from 270 degrees, or from 280 degrees, or from 290 degrees, or from 300 degrees, or from 310 degrees, or from 320 degrees, or from 330 degrees, or from 340 degrees, or from 350 degrees to less than 360 degrees; or from greater than 0 degrees to 10 degrees, or to 20 degrees, or to 30 degrees, or to 40 degrees, or to 50 degrees, or 60 degrees, or to 70 degrees, or to 80 degrees, or to 90 degrees, or to 100 degrees, or to 110 degrees, or to 120 degrees, or to 130 degrees, or to 140 degrees, or to 150 degrees, or to 160 degrees, or to 170 degrees, or to 180 degrees, or to 190 degrees, or to 200 degrees, or to 210 degrees, or to 220 degrees, or to 230 degrees, or to 240 degrees, or to 250 degrees, or to 260 degrees, or to 270 degrees, or to 280 degrees, or to 290 degrees, or to 300 degrees, or to 310 degrees, or to 320 degrees, or to 330 degrees, or to 340 degrees, or to 350 degrees, or to greater than 360 degrees; or any other range of from one of the above minima to one of the above maxima.

Referring to FIG. **2****,** an example of a drainage catheter **200** is illustrated. Drainage catheter **200** has a mid-bore catheter size, for example a 14 French catheter size. Drainage catheter **200** includes a tubular body that extends longitudinally from proximal portion **210** to distal portion **202.** Distal portion **202** includes tapered distal tip **208.** Drainage catheter **200** includes an outer surface and a lumen extending longitudinally throughout drainage catheter **200** from proximal portion **210** to distal tip **208.** As illustrated in FIG. **2****,** distal portion **202** may include a degree of curvature in a longitudinal segment of distal portion **202** such that distal tip **208** may at least approximate the outer surface of drainage catheter **200** proximal to distal tip **208.** Distal tip **208** may confront the outer surface of drainage catheter **200** proximal to distal tip **208.** As a consequence of the degree of curvature in the longitudinal segment of distal portion **202,** a first side of the outer surface of drainage catheter **200** corresponds to an inner curvature, and a second side of the outer surface of drainage catheter **200** opposite to the first side corresponds to an outer curvature of the degree of curvature. As illustrated in FIG. **2****,** the first side faces towards proximal portion **210** of drainage catheter **200** and the second side faces away from proximal portion **210** of drainage catheter.

The first side of the outer surface of drainage catheter **200** includes a plurality of holes **206** spaced apart longitudinally about the inner curvature of distal portion **202.** The second side of distal portion **202** includes elliptically-shaped hole **204** proximal to the most proximal of plurality of holes **206.**

Distal portion **202** may include a material having a defined shape and density. Distal portion **202** may be, or include, a material that is an output of a conveying system including distributive mixing and/or a die system. Distal portion **202** may be, in whole or in part, made up of a flexible base thermoplastic material, such as ultrathane, including particulate additive(s) incorporated into and/or distributed uniformly within the thermoplastic material.

Referring to FIG. **3****,** a perspective view of yet another example of a drainage catheter **300** is illustrated. Drainage catheter **300** has a large-bore catheter size, for example a 24 French catheter size. Drainage catheter **300** includes a tubular body that extends longitudinally from proximal portion **308** to distal portion **302** and has a degree of curvature of 0 degrees. Distal portion **302** includes tapered distal tip **306.** Drainage catheter **300** includes an outer surface and a lumen extending longitudinally throughout drainage catheter **300.** Proximal to distal tip **306** in distal portion **302,** a plurality of holes **304** are spaced apart longitudinally. Plurality of holes **304** may be spaced apart longitudinally and circumferentially to provide a spiral configuration of plurality of holes **304** about the circumference of distal portion **302.** Plurality of holes **304** may be spaced apart circumferentially at 120 degree increments about the circumference of the outer surface of distal portion **302.** The spiral configuration of plurality of holes **304** reduces the chance of one or more of plurality of holes **304** becoming blocked by organs or tissue.

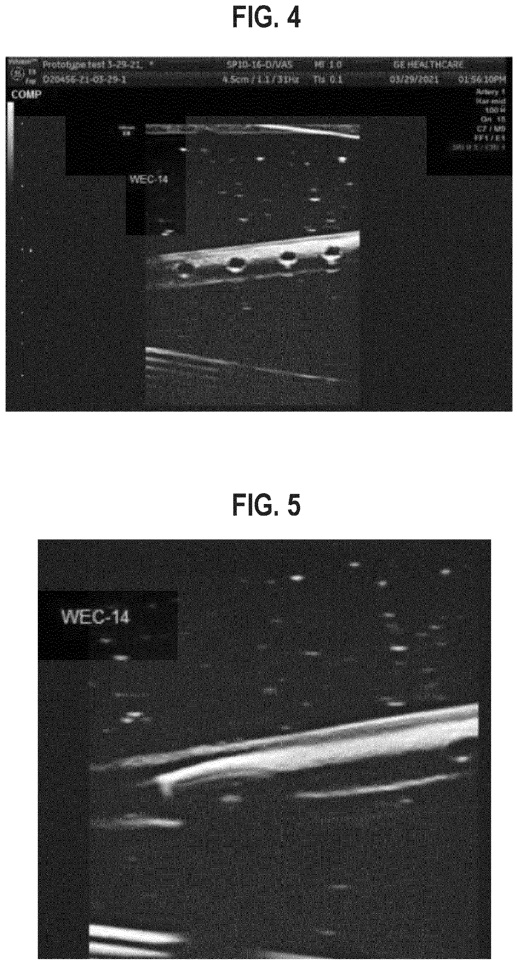

Referring to FIG. **4****,** an ultrasound of an example of a drainage catheter, upon insertion into a phantom pleural cavity filled with fluid, is illustrated. Upon insertion, some of the plurality of holes of the distal portion are visible under ultrasound in relative contrast. Due to the echogenicity of the example of the drainage catheter shown under ultrasound in FIG. **4****,** some of the plurality of holes are very clearly visible under ultrasound, demonstrating the echogenic capability of the drainage catheters according to the principles of the present disclosure, which is not demonstrated by currently available drainage catheters. Currently, physicians use movement or fluid flow or tiny air bubbles to visualize the drainage catheter interface or holes in the drainage catheter of the state of the art. The ultrasound of the drainage catheter illustrated in FIG. **4** includes primarily large particle tungsten particulate additive incorporated into the marker band of the distal portion.

Referring to FIG. **5****,** an ultrasound of an example of a drainage catheter, upon insertion into a phantom pleural cavity filled with fluid, is illustrated. Upon insertion, the tapered distal tip of the distal portion is clearly visible under ultrasound in relative contrast. Further, the most distal of the plurality of holes is very clearly visible on the right side of FIG. **5****,** demonstrating the echogenic capability of the drainage catheters according to the principles of the present disclosure. The ultrasound of the drainage catheter illustrated in FIG. **5** includes primarily large particle tungsten particulate additive incorporated into the marker band of the distal portion.

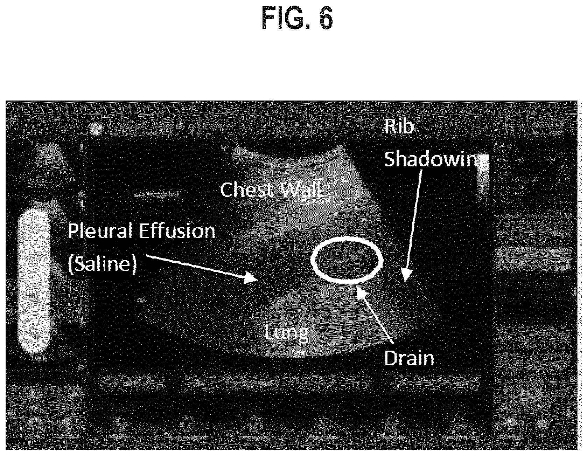

Referring to FIG. **6****,** an ultrasound of a simulated porcine pleural effusion into which another example of a drainage catheter has been inserted, identified, and labeled, is illustrated. The drainage catheter shown under ultrasound in FIG. **6** has a 14 French catheter size. The catheter is shown in FIG. **6** on the ultrasound encircled in white and labeled "Drain."

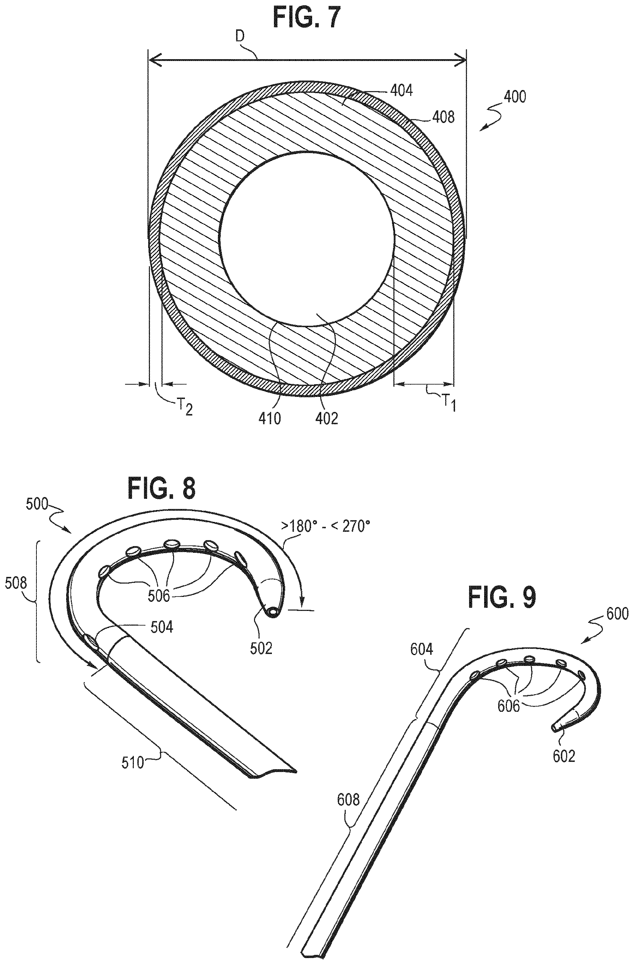

Referring to FIG. **7****,** a longitudinal cross-sectional view of a distal portion of yet another example of a drainage catheter **400** is illustrated. Drainage catheter **400** includes lumen **402** with lumen surface **410.** Drainage catheter **400** has an outer diameter of *D,* where *D* may have a value of from 1.6 millimeters to 12.0 millimeters. The outer diameter *D* of drainage catheter **400** may have a value of from 1.6 millimeters, or from 1.7 millimeters, or from 1.8 millimeters, or from 1.9 millimeters, or from 2.0 millimeters, or from 2.1 millimeters, or from 2.2 millimeters, or from 2.3 millimeters, or from 2.4 millimeters, or from 2.5 millimeters, or from 2.6 millimeters, or from 2.7 millimeters, or from 2.8 millimeters, or from 2.9 millimeters, or from 3.0 millimeters, or from 3.1 millimeters, or from 3.2 millimeters, or from 3.3 millimeters, or from 3.4 millimeters, or from 3.5 millimeters, or from 3.6 millimeters, or from 3.7 millimeters, or from 3.8 millimeters, or from 3.9 millimeters, or from 4.0 millimeters, or from 4.1 millimeters, or from 4.2 millimeters, or from 4.3 millimeters, or from 4.4 millimeters, or from 4.5 millimeters, or from 4.6 millimeters, or from 4.7 millimeters, or from 4.8 millimeters, or from 4.9 millimeters, or from 5.0 millimeters, or from 5.1 millimeters, or from 5.2 millimeters, or from 5.3 millimeters, or from 5.4 millimeters, or from 5.5 millimeters, or from 5.6 millimeters, or from 5.7 millimeters, or from 5.8 millimeters, or from 5.9 millimeters, or from 6.0 millimeters, or from 6.1 millimeters, or from 6.2 millimeters, or from 6.3 millimeters, or from 6.4 millimeters, or from 6.5 millimeters, or from 6.7 millimeters, or from 6.8 millimeters, or from 6.9 millimeters, or from 7.0 millimeters, or from 7.1 millimeters, or from 7.2 millimeters, or from 7.3 millimeters, or from 7.4 millimeters, or from 7.5 millimeters, or from 7.6 millimeters, or from 7.7 millimeters, or from 7.8 millimeters, or from 7.9 millimeters, or from 8.0 millimeters, or from 8.1 millimeters, or from 8.2 millimeters, or from 8.3 millimeters, or from 8.4 millimeters, or from 8.5 millimeters, or from 8.6 millimeters, or from 8.7 millimeters, or from 8.8 millimeters, or from 8.9 millimeters, or from 9.0 millimeters, or from 9.1 millimeters, or from 9.2 millimeters, or from 9.3 millimeters, or from 9.4 millimeters, or from 9.5 millimeters, or from 9.6 millimeters, or from 9.7 millimeters, or from 9.8 millimeters, or from 9.9 millimeters, or from 10.0 millimeters, or from 10.1 millimeters, or from 10.2 millimeters, or from 10.3 millimeters, or from 10.4 millimeters, or from 10.5 millimeters, or from 10.6 millimeters, or from 10.7 millimeters, or from 10.8 millimeters, or from 10.9 millimeters, or from 11.0 millimeters, or from 11.1 millimeters, or from 11.2 millimeters, or from 11.3 millimeters, or from 11.4 millimeters, or from 11.5 millimeters, or from 11.6 millimeters, or from 11.7 millimeters, or from 11.8 millimeters, or from 11.9 millimeters to 12.0 millimeters; or from 1.6 millimeters to 1.7 millimeters, or to 1.8 millimeters, or to 1.9 millimeters, or to 2.0 millimeters, or to 2.1 millimeters, or to 2.2 millimeters, or to 2.3 millimeters, or to 2.4 millimeters, or to 2.5 millimeters, or to 2.6 millimeters, or to 2.7 millimeters, or to 2.8 millimeters, or to 2.9 millimeters, or to 3.0 millimeters, or to 3.1 millimeters, or to 3.2 millimeters, or to 3.3 millimeters, or to 3.4 millimeters, or to 3.5 millimeters, or to 3.6 millimeters, or to 3.7 millimeters, or to 3.8 millimeters, or to 3.9 millimeters, or to 4.0 millimeters, or to 4.1 millimeters, or to 4.2 millimeters, or to 4.3 millimeters, or to 4.4 millimeters, or to 4.5 millimeters, or to 4.6 millimeters, or to 4.7 millimeters, or to 4.8 millimeters, or to 4.9 millimeters, or to 5.0 millimeters, or to 5.1 millimeters, or to 5.2 millimeters, or to 5.3 millimeters, or to 5.4 millimeters, or to 5.5 millimeters, or to 5.6 millimeters, or to 5.7 millimeters, or to 5.8 millimeters, or to 5.9 millimeters, or to 6.0 millimeters, or to 6.1 millimeters, or to 6.2 millimeters, or to 6.3 millimeters, or to 6.4 millimeters, or to 6.5 millimeters, or to 6.6 millimeters, or to 6.7 millimeters, or to 6.8 millimeters, or to 6.9 millimeters, or to 7.0 millimeters, or to 7.1 millimeters, or to 7.2 millimeters, or to 7.3 millimeters, or to 7.4 millimeters, or to 7.5 millimeters, or to 7.6 millimeters, or to 7.7 millimeters, or to 7.8 millimeters, or to 7.9 millimeters, or to 8.0 millimeters, or to 8.1 millimeters, or to 8.2 millimeters, or to 8.3 millimeters, or to 8.4 millimeters, or to 8.5 millimeters, or to 8.6 millimeters, or to 8.7 millimeters, or to 8.8 millimeters, or to 8.9 millimeters, or to 9.0 millimeters, or to 9.1 millimeters, or to 9.2 millimeters, or to 9.3 millimeters, or to 9.4 millimeters, or to 9.5 millimeters, or to 9.6 millimeters, or to 9.7 millimeters, or to 9.8 millimeters, or to 9.9 millimeters, or to 10.0 millimeters, or to 10.1 millimeters, or to 10.2 millimeters, or to 10.3 millimeters, or to 10.4 millimeters, or to 10.5 millimeters, or to 10.6 millimeters, or to 10.7 millimeters, or to 10.8 millimeters, or to 10.9 millimeters, or to 11.0 millimeters, or to 11.1 millimeters, or to 11.2 millimeters, or to 11.3 millimeters, or to 11.4 millimeters, or to 11.5 millimeters, or to 11.6 millimeters, or to 11.7 millimeters, or to 11.8 millimeters, or to 11.9 millimeters, or to 12.0 millimeters; or from any one of the above minima to any one of the above maxima. In other examples, the outer diameter *D* of drainage catheter **400** may preferably have a value of from 1.6 millimeters to 10.0 millimeters.

The distal portion of drainage catheter **400** includes inner layer **404** made of base ultrathane polymeric material. Inner layer **404** has a thickness *T*₁ and outer layer **408** has a thickness *T*₂*.* The combined thickness (*T*₁ + *T*₂) of inner layer **404** and outer layer **408** may have a value of from 0.2 millimeters to 1.6 millimeters, or from 0.3 millimeters, or from 0.4 millimeters, or from 0.5 millimeters, or from 0.6 millimeters, or from 0.7 millimeters, or from 0.8 millimeters, or from 0.9 millimeters, or from 1.0 millimeters, or from 1.1 millimeters, or from 1.2 millimeters, or from 1.3 millimeters, or from 1.4 millimeters, or from 1.5 millimeters to 1.6 millimeters; or from 0.2 millimeters to 0.3 millimeters, or to 0.4 millimeters, or to 0.5 millimeters, or to 0.6 millimeters, or to 0.7 millimeters, or to 0.8 millimeters, or to 0.9 millimeters, or to 1.0 millimeters, or to 1.1 millimeters, or to 1.2 millimeters, or to 1.3 millimeters, or to 1.4 millimeters, or to 1.5 millimeters, or to 1.6 millimeters; or from any one of the above minima to any one of the above maxima. The combined thickness (*T*₁ + *T*₂) of inner layer **404** and outer layer **408** may preferably have a thickness of from 0.2 millimeters to 1.2 millimeters.

Outer layer **408** is made up of thermoplastic material, such as ultrathane, including particulate additive(s) incorporated into the thermoplastic material. Inner layer **404** may be made of base ultrathane polymeric material. The outer layer may be made up of thermoplastic material, such as ultrathane, including particulate additive(s) incorporated into the thermoplastic material. Accordingly, either an inner surface of the layer made of base ultrathane polymeric material or an outer surface of the layer made of base ultrathane polymeric material may be covered by the layer including particulate additive(s).

One layer of inner layer **404** and outer layer **408** may account for from 5% to 30% of the overall combined thickness (*T*₁ and *T*₂) of inner layer **404** and outer layer **408,** or from 6%, or from 7%, or from 8%, or from 9%, or from 10%, or from 11%, or from 12%, or from 13%, or from 14%, or from 15%, or from 16%, or from 17%, or from 18%, or from 19%, or from 20%, or from 21%, or from 22%, or from 23%, or from 24%, or from 25%, or from 26%, or from 27%, or from 28%, or from 29% to 30%; or from 5% to 6%, or to 7%, or to 8%, or to 9%, or to 10%, or to 11%, or to 12%, or to 13%, or to 14%, or to 15%, or to 16%, or to 17%, or to 18%, or to 19%, or to 20%, or to 21%, or to 22%, or to 23%, or to 24%, or to 25%, or to 26%, or to 27%, or to 28%, or to 29%, or to 30%, or from any one of the above minima percentages to any one of the above maxima percentages. One layer of inner layer **404** and outer layer **408** may preferably account for from 10% to 20% of the overall combined thickness (*T*₁ and *T*₂) of inner layer **404** and outer layer **408.** The other layer of inner layer **404** and outer layer **408** may account for the difference between the percentage of the one layer of the overall combined thickness (*T*₁ and *T*₂) of inner layer **404** and outer layer **408** and 100% overall combined thickness (*T*₁ and *T*₂) of inner layer **404** and outer layer **408,** or from 70% to 95% of the overall combined thickness, or from 71%, or from 72%, or from 73%, or from 74%, or from 75%, or from 76%, or from 77%, or from 78%, or from 79%, or from 80%, or from 81%, or from 82%, or from 83%, or from 84%, or from 85%, or from 86%, or from 87%, or from 88%, or from 89%, or from 90%, or from 91%, or from 92%, or from 93%, or from 94% to 95%; or from 70% to 71%, or to 72%, or to 73%, or to 74%, or to 75%, or to 76%, or to 77%, or to 78%, or to 79%, or to 80%, or to 81%, or to 82%, or to 83%, or to 84%, or to 85%, or to 86%, or to 87%, or to 88%, or to 89%, or to 90%; or from any one of the above minima to any one of the above maxima. Preferably, the other layer of inner layer **404** and outer layer **408** may account for from 80% to 90% overall combined thickness (*T*₁ and *T*₂) of inner layer **404** and outer layer **408.**

Referring to FIG. **8****,** a perspective view of a distal portion of yet another example of a drainage catheter **500** is illustrated. Drainage catheter **500** includes distal portion **508** and proximal portion **510.** Distal portion **508** includes a degree of curvature of greater than 180 degrees and less than 270 degrees. Plurality of holes **506** are spaced apart longitudinally on the inner curvature of distal portion **508** between tapered distal tip **502** and proximal portion **510.** Elliptically-shaped hole **504** is on the outer curvature of distal portion **508** proximal to the most proximal of plurality of holes **506.** Distal portion **508** includes at least an outer layer that includes a thermoplastic material including or incorporating particulate additive(s).

Referring to FIG. **9****,** a perspective view of a distal portion of yet another example of a drainage catheter **600** is illustrated. Drainage catheter **600** includes proximal portion **608,** and distal portion **604** including plurality of holes **606** on the inner curvature of distal portion **604** and tapered distal tip **602.** Drainage catheter **600** may be of a smaller bore catheter size than drainage catheter **500.** Distal portion **604** includes at least an outer layer that includes a thermoplastic material including or incorporating particulate additive(s).

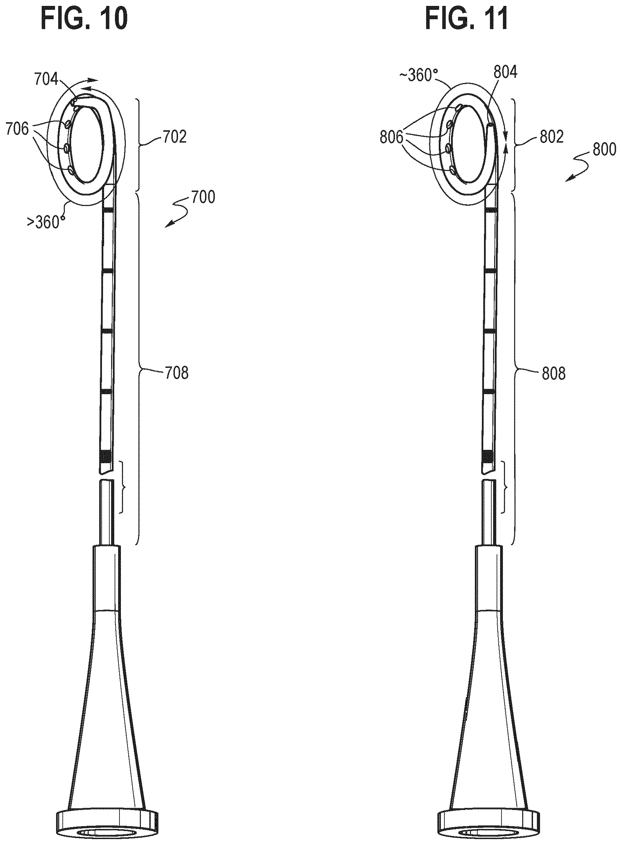

Referring to FIG. **10****,** a perspective view of yet another example of a drainage catheter **700,** with a distal portion **702** with a degree of curvature of greater than 360 degrees, is illustrated. Drainage catheter **700** includes distal portion **702** and proximal portion **708.** Distal portion **702** includes a circular loop in a longitudinal segment of distal portion **702** (i.e., "pigtail" configuration) such that distal tip **704** at least approximates, and may confront, the outer surface of drainage catheter **700** proximal to tapered distal tip **704.** As a consequence of the circular loop in the longitudinal segment of distal portion **702,** a first side of the outer surface of drainage catheter **700** corresponds to an inner circumference of the circular loop, on which plurality of holes **706** are spaced apart longitudinally about the inner circumference of the circular loop. A second side of the outer surface of drainage catheter **700,** opposite to the first side, corresponds to an outer circumference of the circular loop, on which the elliptically-shaped hole (not shown) is located. Placement of the elliptically-shaped hole on the outer circumference of the circular loop results in the elliptically-shaped hole being opposite to the direction of the circular loop of distal portion **702,** which makes trocar insertion easier.

Referring to FIG. **11****,** a perspective view of yet another example of a drainage catheter **800,** with a distal portion **802** with a degree of curvature of about 360 degrees, is illustrated. Drainage catheter **800** includes distal portion **802** and proximal portion **808.** Distal portion **802** includes a circular loop in a longitudinal segment of distal portion **802** such that distal tip **804** at least approximates, and may confront, the outer surface of drainage catheter **800** proximal to tapered distal tip **804.** As a consequence of the circular loop in the longitudinal segment of distal portion **802,** a first side of the outer surface of drainage catheter **800** corresponds to an inner circumference of the circular loop, on which plurality of holes **806** are spaced apart longitudinally about the inner circumference of the circular loop. A second side of the outer surface of drainage catheter **800,** opposite to the first side, corresponds to an outer circumference of the circular loop, on which the elliptically-shaped hole (not shown) is located.

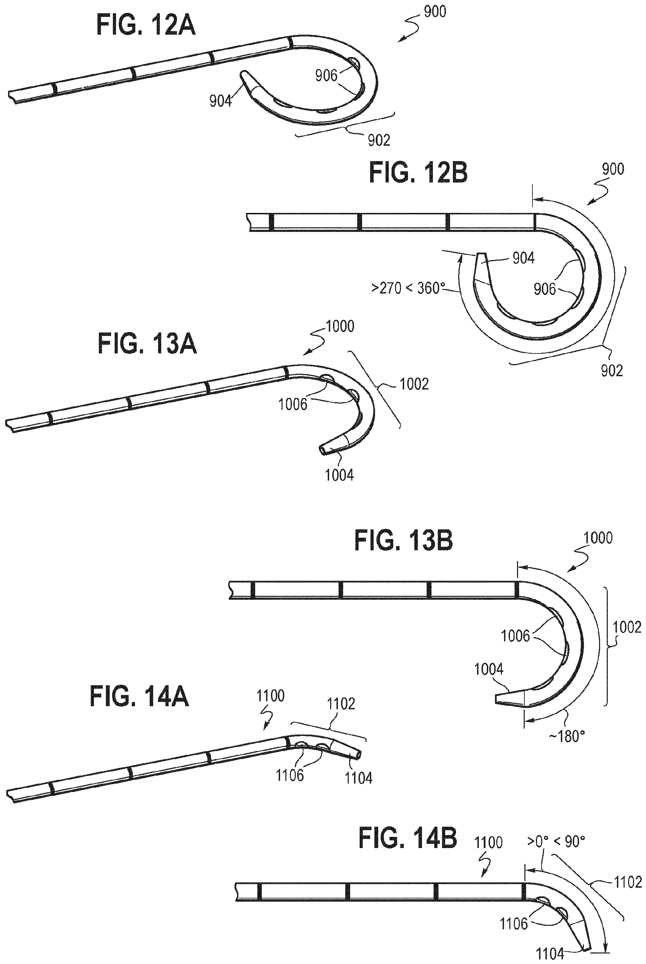

Referring to FIG. **12A****,** a perspective view of a distal portion **902** of yet another example of a drainage catheter **900** is illustrated. FIG. **12B** illustrates a side view of distal portion **902,** with a degree of curvature in distal portion **902** of greater than 270 degrees and less than 360 degrees between the proximal portion and distal tip **904.** Distal tip **904** at least approximates the outer surface of drainage catheter **900.** The inner curvature of distal portion **902** includes plurality of holes **906** spaced apart longitudinally. Drainage catheter **900** may resemble the shape of a "shepherd's hook."

Referring to FIG. **13A****,** a perspective view of a distal portion **1002** of yet another example of a drainage catheter **1000** is illustrated. FIG. **13B** illustrates a side view of distal portion **1002,** with a degree of curvature in distal portion **1002** of about 180 degrees between distal tip **1004** and the proximal portion. The inner curvature of distal portion **1002** includes plurality of holes **1006** spaced apart longitudinally. Drainage catheter **1000** may resemble the shape of a "candy cane."

Referring to FIG. **14A****,** a perspective view of a distal portion **1102** of yet another example of a drainage catheter **1100** is illustrated. FIG. **14B** illustrates a side view of distal portion **1102,** with a degree of curvature of greater than 0 degrees and less than 90 degrees between distal tip **1104** and the proximal portion. The inner curvature of distal portion **1102** includes plurality of holes **1106** spaced apart longitudinally. Drainage catheter **1100** may resemble the shape of a "hockey stick."

In an example of a drainage catheter, all or part of the drainage catheter may include three concentric layers of material, one layer including particulate additive(s) incorporated into or distributed uniformly within the material. The layer including particulate additive(s) may be a middle layer, with an exterior layer and an interior layer made of ultrathane. It is expected that a three-layer drainage catheter would demonstrate increased echogenicity while disguising or hiding the particulate additive(s) filling and retaining the appearance and mechanical properties of the ultrathane material. In certain examples, the 3-layer arrangement of material is included in the distal portion, from proximal to the most proximal hole to the tapered distal tip of a drainage catheter, which would allow for the entire distal portion to be visible under ultrasound. In other examples, the 3-layer arrangement of material is included throughout the entire drainage catheter, which would allow for the entire length of the drainage catheter to be visible under ultrasound. Further, it is expected that the plurality of holes may appear more pronounced under ultrasound.

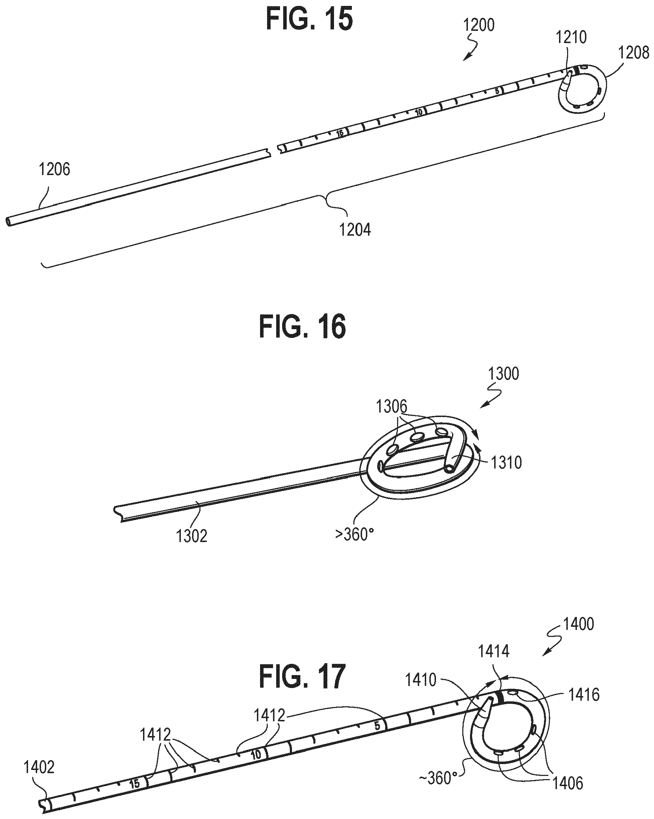

Referring to FIG. **15****,** yet another example of a drainage catheter **1200** is illustrated. Drainage catheter **1200** includes a tubular body that extends longitudinally from proximal portion **1206** to distal portion **1208.** Distal portion **1208** includes tapered distal tip **1210.** Drainage catheter **1200** includes an outer surface and a lumen extending longitudinally throughout drainage catheter **1200** from proximal portion **1206** to distal tip **1210.** Drainage catheter **1200** has a longitudinal cross-sectional outer diameter. The lumen extending longitudinally throughout drainage catheter **1200** has a longitudinal cross-sectional inner diameter that is shorter than the longitudinal cross-sectional diameter outer diameter of drainage catheter **1200.** As illustrated in FIG. **15****,** distal portion **1208** may include a circular loop in a longitudinal segment of distal portion **1208** (i.e., "pigtail" configuration) such that distal tip **1210** at least approximates the outer surface of drainage catheter **1200** proximal to distal tip **1210.** The distal tip **1210** may confront the outer surface of drainage catheter **1200** proximal to distal tip **1210.** As a consequence of the circular loop in the longitudinal segment of distal portion **1208,** a first side of the outer surface of drainage catheter **1200** corresponds to an inner circumference of the circular loop, and a second side of the outer surface of drainage catheter **1200** opposite to the first side corresponds to an outer circumference of the circular loop. Distal tip **1210** may be hardened relative to flexibility of drainage catheter **1200** so as to be configured for increased ease of penetration and/or insertion, and/or increased accuracy of placement of drainage catheter **1200.**

Drainage catheter **1200** includes a flexible base thermoplastic material selected from a group consisting of a polyester elastomer, a polyurethane polymer, a polyamide elastomer, and a polyether block amide. The base thermoplastic material includes a particulate additive configured to introduce echopacity and/or echogenicity to drainage catheter **1200.** In certain examples, the base thermoplastic material may include two or more particulate additives.

In certain examples, a particulate additive may include glass microspheres. In other examples, a particulate additive may include titanium dioxide-coated hollow glass spheres. In still other examples, a particulate additive may include monodisperse silica microspheres. In still other examples, a particulate additive may include polyethylene nanospheres. In still other examples, a particulate additive may include zinc oxide-coated hollow glass microspheres. In still other examples, a particulate additive may include silver-coated hollow glass microspheres. In still other examples, a particulate additive may include retroreflective aluminum-coated solid barium titanate glass microspheres. In particularly preferred examples, a particulate additive may include glass microspheres.

In the above examples of particulate additives including spheres, microspheres, or nanospheres, a fill rate of a particulate additive may be consistent at a fixed additive-to-polymer volume ratio but may vary in weight %. In certain examples, the weight % of the particulate additive may be from about 1 weight %, or from about 2 weight %, or from about 3 weight %, or from about 4 weight %, or from about 5 weight %, to about 10 weight %; or from about 5 weight % to about 6 weight %, or to about 7 weight %, or to about 8 weight %, or to about 9 weight %; or any other range of from about one of the above minima to about one of the above maxima. In particularly preferred examples, glass microspheres may be included as a particulate additive in about 3 weight %, about 4 weight %, or about 5 weight %.

It is expected that particle size and shape of a particulate additive may be critical to efficacy of drainage catheter **1200** to be visible through X-ray and/or ultrasound. In certain examples where a particulate additive includes glass microspheres, the glass microspheres may have a diameter (±5 microns) of from about 6 microns, or from about 10 microns, or from about 20 microns, or from about 30 microns, or from about 40 microns, or from about 50 microns, or from about 60 microns, or from about 70 microns, or from about 80 microns, or from about 90 microns, or from about microns, to about 250 microns; or from about 6 microns to about 20 microns, or to about 30 microns, or to about 40 microns, or to about 50 microns, or to about 60 microns, or to about 70 microns, or to about 80 microns, or to about 90 microns, or to about 100 microns, or to about 110 microns, or to about 120 microns, or to about 130 microns, or to about 140 microns, or to about 150 microns, or to about 160 microns, or to about 170 microns, or to about 180 microns, or to about 190 microns, or to about 200 microns, or to about 210 microns, or to about 220 microns, or to about 230 microns, or to about 240 microns; or any other range of from about one of the above minima to about one of the above maxima. In particularly preferred examples, a particulate additive may include glass microspheres with a diameter (±5 microns) of from about 6 microns to about 20 microns. In certain examples where a particulate additive includes tungsten in particulate form and/or tungsten carbide, the tungsten in particulate form and/or tungsten carbide may have a diameter preferably from about 2 microns to about 200 microns, more preferably from about 20 to about 50 microns, and most preferably from about 10 to about 20 microns, including from about 4 microns, or from about 6 microns, or from about 8 microns, or from about 10 microns, or from about 12 microns, or from about 14 microns, or from about 16 microns, or from about 18 microns, or from about 20 microns, to about 200 microns; or from about 2 microns to about 10 microns, or to about 12 microns, or to about 14 microns, or to about 16 microns, or to about 18 microns, or to about 20 microns, or to about 30 microns, or to about 40 microns, or to about 50 microns, or to about 60 microns, or to about 70 microns, or to about 80 microns, or to about 90 microns, or to about 100 microns, or to about 110 microns, or to about 120 microns, or to about 130 microns, or to about 140 microns, or to about 150 microns, or to about 160 microns, or to about 170 microns, or to about 180 microns, or to about 190 microns; or any other range of from about one of the above minima to about one of the above maxima.

In certain examples, drainage catheter **1200** may include a tubular body including an inner longitudinal layer covered by a smooth outer layer of non-echogenic material.

Referring now to FIG. **16****,** a perspective view of a distal portion **1300** of an example of a drainage catheter **1302** is illustrated. Distal portion **1300** includes a circular loop in a longitudinal segment of distal portion **1300** (i.e., "pigtail" configuration) such that tapered distal tip **1310** at least approximates, and may confront, the outer surface of drainage catheter **1302** proximal to distal tip **1310.** Distal tip **1310** may be a marker band including a particulate additive. The degree of curvature in distal portion **1300** is greater than 360 degrees. The circular loop of distal portion **1300** aids in drainage performance of drainage catheter **1302.** The circular loop minimizes the linear footprint of drainage catheter **1302** in the pleural space. The circular loop also anchors drainage catheter **1302** in a correct location, thereby reducing the chance of displacement or removal of drainage catheter **1302** from the pleural space. The circular loop also provides a landmark when imaging under ultrasound and fluoroscopy. As a consequence of the circular loop in the longitudinal segment of distal portion **1308,** a first side of the outer surface of drainage catheter **1302** corresponds to an inner circumference of the circular loop, on which a plurality of holes **1306** are spaced apart longitudinally about the inner circumference of the circular loop (only four of the at least six holes **1306** are shown in FIG. **16**). Each of the plurality of holes **1306** may have a width corresponding to about a diameter of the lumen extending longitudinally throughout drainage catheter **1302.** Positioning the plurality of holes **1306** on the inner circumference of the circular loop reduces the probability of one or more of the plurality of holes **1306** becoming blocked by an organ or tissue. The plurality of holes **1306** may include 2 holes, 3 holes, 4 holes, 5 holes, 6 holes, 7 holes, 8 holes, 9 holes, 10 holes, or more than 10 holes.

Referring now to FIG. **17****,** a perspective view of a distal portion **1400** of another example of a drainage catheter **1402** is illustrated. Distal portion **1400** includes a circular loop in a longitudinal segment of a distal portion **1400** (i.e., "pigtail" configuration) such that distal tip **1410** at least approximates, and may confront, the outer surface of drainage catheter **1402** proximal to tapered distal tip **1410.** As a consequence of the circular loop in the longitudinal segment of distal portion **1400,** a first side of the outer surface of drainage catheter **1402** corresponds to an inner circumference of the circular loop, on which a plurality of holes **1406** are spaced apart longitudinally about the inner circumference of the circular loop (only three of the plurality of holes **1406** are shown in FIG. **17**). The degree of curvature in distal portion **1400** is approximately 360 degrees. A second side of the outer surface of drainage catheter **1402** opposite to the first side corresponds to an outer circumference of the circular loop, on which elliptically-shaped hole **1416** is located. Elliptically-shaped hole **1416** may be configured for insertion of a trocar device. Placement of elliptically-shaped hole **1416** on the outer circumference of the circular loop such that elliptically-shaped hole **1416** consequently results in elliptically-shaped hole **1416** being opposite to the direction of the circular loop of distal portion **1400,** which makes trocar insertion easier. Elliptically-shaped hole **1416** is also easily identifiable under ultrasound because of the unique shape of the elliptically-shaped hole **1416.** Elliptically-shaped hole **1416** is an important landmark, because it is the most proximal hole of drainage catheter **1402,** and assures the user of drainage catheter **1402** that all holes of drainage catheter **1402** are in the pleural space. Elliptically-shaped hole **1416** may be used for the "rapid exchange" Seldinger technique, permitting use of a shorter wire guide (as the wire does not have to traverse the length of drainage catheter **1402).** Elliptically-shaped hole **1416** also increases hub options because the wire does not have to traverse the hub. Elliptically-shaped hole **1416** must be large enough to accommodate a straightening stiffener, which may have a slightly smaller outer diameter than the inner diameter of drainage catheter **1402;** accordingly, the elliptical shape of elliptically-shaped hole **1416** is particularly important, and the major axis of the ellipse should be parallel to the longitudinal axis of drainage catheter **1402.** The elliptically-shaped hole **1416** must have a minor axis larger than the maximum diameter of each of the plurality of holes **1406.**

Proximal to elliptically-shaped hole **1416** on the circular loop is marker band **1414** external to and circumferentially encircling the outer surface of drainage catheter **1402.** Marker band **1414** may be at least 5 millimeters wide in the longitudinal (proximal-to-distal) direction and may be configured to provide visual confirmation to an operator of a depth of drainage catheter **1402** in a patient past a most proximal hole of plurality of holes **1406.** It is expected that marker band **1414** may beneficially allow clinicians to avoid blind insertion of catheters while providing a more accurate indication of depth relative to the most proximal of the plurality of holes **1406.** In certain examples, drainage catheter **1402** may have more than one marker band **1414** spaced apart longitudinally and proximal to the most proximal of the plurality of holes **1406.** Proximal to marker band **1414,** drainage catheter **1402** includes graduated depth indicator lines **1412** incrementally spaced apart longitudinally and configured to provide optical perception of insertion depth of drainage catheter **1402** into a patient. Graduated depth indicator lines **1412** provide precise insertion control and facilitate post-operative maintenance. Optimizing the number of the plurality of holes **1406** may maintain the strength of drainage catheter **1402** without sacrificing drainage performance. Marker band **1414** is visible under ultrasound and fluoroscopy, which may allow physicians to visualize marker band **1414,** which confirms that all of the plurality of holes **1406** and the elliptically-shaped hole **1416** are in the body, which is critical for drainage. Distal tip **1410** may be a marker band.

Marker band **1414** and distal tip **1410** may include material(s) configured to provide drainage catheter **1402** with desirable echopacity and/or echogenicity. The drainage catheters of the present invention exhibit increased echogenicity compared to drainage catheters currently on the market. The drainage catheters of the present invention also have enhanced radiopacity, because marker band **1414** and distal tip **1410** are distinguishable from the remainder of drainage catheter **1402** under fluoroscopy. In certain examples, marker band **1414** may include barium sulfate. In other examples, marker band **1414** may include bismuth oxalate. In still other examples, marker band **1414** may include bismuth oxychloride. In still other examples, marker band **1414** may include bismuth sulfate. In still other examples, marker band **1414** may include tungsten in particulate form. In still other examples, marker band **1414** may include tungsten carbide. In still other examples, marker band **1414** may include aluminum silicate particles. In still other examples, marker band **1414** may include calcium carbonate. In still other examples, marker band **1414** may include calcium hydroxide, and/or a hydrate thereof. In still other examples, marker band **1414** may include calcium oxide or quicklime. In still other examples, marker band **1414** may include calcium sulfate. In still other examples, marker band **1414** may include clay. In still other examples, marker band **1414** may include gypsum. In still other examples, marker band **1414** may include mica. In still other examples, marker band **1414** may include muscovite mica. In still other examples, marker band **1414** may include phlogopite mica. In still other examples, marker band **1414** may include micacious iron oxide. In still other examples, marker band **1414** may include talc. Marker band **1414** may be configured to provide drainage catheter **1402** with desired properties of echopacity and/or echogenicity. Marker band **1414** may allow for a more concentrated fill percentage of echopaque, echogenic, and/or radiopaque materials. A fill rate of material(s) in marker band **1414** may be in a weight % of from about 10 weight %, or from about 15 weight %, or from about 20 weight %, or from about 25 weight %, or from about 30 weight %, or from about 35 weight %, or from about 40 weight %, or from about 45 weight %, or from about 50 weight %, from about 55 weight %, or from about 60 weight % to about 90 weight %; or from about 10 weight % to about 40 weight %, or to about 45 weight %, or to about 50 weight %, or to about 55 weight %, or to about 60 weight %, or to about 65 weight %, or to about 70 weight %, or to about 75 weight %, or to about 80 weight %, or to about 85 weight; or any other range of from about one of the above minima to about one of the above maxima. In particularly preferred examples, marker band **1414** may include a tungsten species in from about 70 weight % to about 90 weight %. In other particularly preferred examples, marker band **1414** may include a bismuth species in from about 40 weight % to about 60 weight %.

Distal tip **1410** may include material(s) configured to provide drainage catheter **1402** with desirable echopacity and/or echogenicity. In certain examples, distal tip **1410** may include barium sulfate. In other examples, distal tip **1410** may include bismuth oxychloride. In still other examples, distal tip **1410** may include tungsten in particulate form. In still other examples, distal tip **1410** may include tungsten carbide. In still other examples, distal tip **1410** may include aluminum silicate particles. In still other examples, distal tip **1410** may include calcium carbonate. In still other examples, distal tip **1410** may include calcium hydroxide, and/or a hydrate thereof. In still other examples, distal tip **1410** may include calcium oxide or quicklime. In still other examples, distal tip **1410** may include calcium sulfate. In still other examples, distal tip **1410** may include clay. In still other examples, distal tip **1410** may include gypsum. In still other examples, distal tip **1410** may include mica. In still other examples, distal tip **1410** may include muscovite mica. In still other examples, distal tip **1410** may include phlogopite mica. In still other examples, distal tip **1410** may include micacious iron oxide. In still other examples, distal tip **1410** may include talc. Distal tip **1410** may be configured to provide drainage catheter **1402** with desired properties of echopacity and/or echogenicity. Distal tip **1410** may allow for a more concentrated fill percentage of echopaque and/or echogenic materials. A fill rate of material(s) in distal tip **1410** may be in a weight % of from about 10 weight %, or from about 15 weight %, or from about 20 weight %, or from about 25 weight %, or from about 30 weight %, or from about 35 weight %, or from about 40 weight %, or from about 45 weight %, or from about 50 weight %, or from about 55 weight %, or from about 60 weight % to about 90 weight %, or from about 10 weight % to about 40 weight %, or to about 45 weight %, or to about 50 weight %, or to about 55 weight %, or to about 60 weight %, or to about 65 weight %, or to about 70 weight %, or to about 75 weight %, or to about 80 weight %, or to about 85 weight %, or any other range of from about one of the above minima to about one of the above maxima. In particularly preferred examples, distal tip **1410** may include a tungsten species in from about 70 weight % to about 90 weight %. In other particularly preferred examples, distal tip **1410** may include a bismuth species in from about 40 weight % to about 60 weight %. The material(s) making up distal tip **1410** may be different from the material(s) making up marker band **1414.** Distal tip **1410** is distinguishable from drainage catheter **1402** under ultrasound.

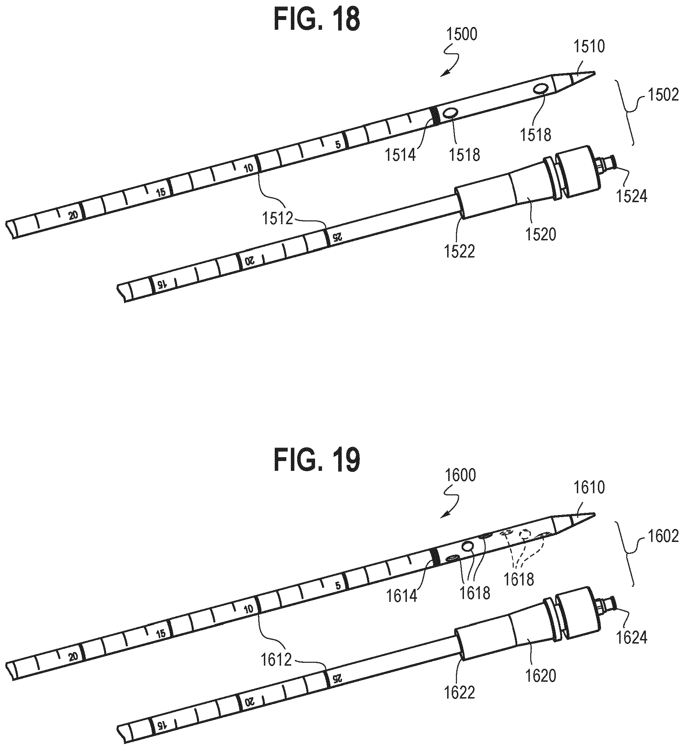

Referring now to FIG. **18****,** a perspective view of yet another example of a drainage catheter **1500** is illustrated. Drainage catheter **1500** includes a tubular body that extends longitudinally from proximal portion **1520** to distal portion **1516** and has a degree of curvature of 0 degrees. Distal portion **1516** includes tapered distal tip **1510.** Drainage catheter **1500** includes an outer surface and a lumen extending longitudinally throughout drainage catheter **1500.** Drainage catheter **1500** has a longitudinal cross-sectional outer diameter. The lumen extending longitudinally throughout drainage catheter **1500** has a longitudinal cross-sectional inner diameter that is shorter than the longitudinal cross-sectional outer diameter of drainage catheter **1500.** Distal tip **1510** may be a marker band and may be hardened relative to flexibility of drainage catheter **1500** so as to be configured for increased ease of penetration and/or insertion, and/or increased accuracy of placement of drainage catheter **1500.** In proximal portion **1516,** marker band **1514** circumferentially encircles the outer surface of drainage catheter **1500** proximal to distal tip **1510.** Proximal to marker band **1514,** drainage catheter **1500** includes graduated depth indicator lines **1512** incrementally spaced apart longitudinally and configured to provide optical perception of insertion depth of drainage catheter **1500** into a patient. Graduated depth indicator lines **1512** provide precise insertion control and facilitate post-operative maintenance. Proximal to distal tip **1510** and distal to marker band **1514,** a plurality of holes **1518** are spaced apart longitudinally (only two of the plurality of holes **1518** are shown in FIG. **18**). In certain examples, drainage catheter **1500** may have more than one marker band **1514** spaced apart longitudinally and proximal to the most proximal of the plurality of holes **1518.** In certain examples, drainage catheter **1500** may have a catheter size of from 6 French to 40 French, including from 7 French, or from 8 French, or from 9 French, or from 10 French, or from 11 French, or from 12 French, or from 13 French, or from 14 French, or from 15 French, or from 16 French, or from 17 French, or from 18 French, or from 19 French, or from 20 French, or from 21 French, or from 22 French, or from 23 French, or from 24 French, or from 25 French, or from 26 French, or from 27 French, or from 28 French, or from 29 French, or from 30 French, or from 31 French, or from 32 French, or from 33 French, or from 34 French, or from 35 French, or from 36 French, or from 37 French, or from 38 French, or from 39 French to 40 French; or from 6 French to 7 French, or to 8 French, or to 9 French, or to 10 French, or to 11 French, or to 12 French, or to 13 French, or to 14 French, or to 15 French, or to 16 French, or to 17 French, or to 18 French, or to 19 French, or to 20 French, or to 21 French, or to 22 French, or to 23 French, or to 24 French, or to 25 French, or to 26 French, or to 27 French, or to 28 French, or to 29 French, or to 30 French, or to 31 French, or to 32 French, or to 33 French, or to 34 French, or to 35 French, or to 36 French, or to 37 French, or to 38 French, or to 39 French; or any other range of from one of the above minima to one of the above maxima.

Referring now to FIG. **19****,** a perspective view of yet another example of a drainage catheter **1600** is illustrated. Drainage catheter **1600** includes a tubular body that extends longitudinally from proximal portion **1620** to distal portion **1616.** Distal portion **1616** includes tapered distal tip **1610.** In distal portion **1616,** marker band **1614** circumferentially encircles the outer surface of drainage catheter **1600** proximal to distal tip **1610.** Proximal to marker band **1614,** drainage catheter **1600** includes graduated depth indicator lines **1612** incrementally spaced apart longitudinally and configured to provide optical perception of insertion depth of drainage catheter **1600** into a patient. Graduated depth indicator lines **1612** provide precise insertion control and facilitate post-operative maintenance. Proximal to distal tip **1610** and distal to marker band **1614,** a plurality of holes **1618** are spaced apart longitudinally and circumferentially to provide a spiral configuration of the plurality of holes **1618** about the circumference of distal portion **1616.** The spiral configuration of the plurality of holes **1618** reduces the chance of one or more of the plurality of holes **1618** becoming blocked by organs or tissue.

In an example, a drainage catheter of the present disclosure includes a tubular body extending along a longitudinal axis, the tubular body including: a lumen extending longitudinally throughout the tubular body; a distal portion including a tapered distal tip; and a marker band encircling the tubular body proximal to the distal tip; wherein the distal portion has a degree of curvature of greater than 0 degrees; wherein the distal portion includes a plurality of holes spaced apart longitudinally, the plurality of holes distal to the marker band and proximal to the distal tip; wherein the distal portion includes an elliptically-shaped hole distal to the marker band, and wherein the marker band and the distal tip are configured to be visible by echogenicity and/or echopacity. The distal portion may include a circular loop in a longitudinal segment of the tubular body such that the distal tip at least approximates an outer surface of the tubular body proximal to the distal tip, the circular loop including an inner circumference and an outer circumference; wherein the inner circumference includes the plurality of holes; and wherein the outer circumference includes the elliptically-shaped hole. The marker band and the distal tip each may include from about 70 weight % to about 90 weight % of tungsten. The marker band and the distal tip each may include from about 40 weight % to about 60 weight % of bismuth. The tubular body may include from about 1 weight % to about 10 weight % of a particulate additive including spheres, microspheres, and/or nanospheres. The particulate additive may include glass microspheres of a particle size of from about 2 microns to about 100 microns. The tubular body may include graduated depth indicator lines spaced apart longitudinally and proximal to the plurality of holes. The marker band may be configured to provide a visual confirmation to an operator of a depth of the drainage catheter in a pleural cavity of a patient past a most proximal hole of the plurality of holes. The marker band may be at least 5 millimeters wide longitudinally.

In another example, a drainage catheter of the present disclosure includes a tubular body extending along a longitudinal axis, the tubular body including: a lumen extending longitudinally throughout the tubular body, the lumen having a cross-sectional inner diameter; a distal portion including a tapered distal tip; and a marker band encircling the tubular body proximal to the distal tip; wherein the distal portion has a degree of curvature of 0 degrees; wherein the distal portion includes a plurality of holes spaced apart longitudinally and circumferentially about the circumference of the distal portion, the plurality of holes distal to the marker band and proximal to the distal tip; and wherein the marker band and the distal tip are configured to be visible by echogenicity and/or echopacity. The marker band and the distal tip each may include from about 70 weight % to about 90 weight % of tungsten. The marker band the distal tip each may include from about 40 weight % to about 60 weight % of bismuth. The tubular body may include from about 1 weight % to about 10 weight % of a particulate additive including spheres, microspheres, and/or nanospheres. The particulate additive may include glass microspheres of a particle size of from about 2 microns to about 100 microns. The tubular body may include graduated depth indicator lines spaced apart longitudinally and proximal to the plurality of holes. The marker band may be configured to provide a visual confirmation to an operator of a depth of the drainage catheter in a pleural cavity of a patient past a most proximal hole of the plurality of holes.

In yet another example, a drainage catheter of the present disclosure includes a tubular body extending along a longitudinal axis, the tubular body including: a lumen extending longitudinally throughout the tubular body; a distal portion including a tapered distal tip; and a marker band encircling the tubular body proximal to the distal tip; wherein the distal portion has a degree of curvature of at least 360 degrees; wherein the distal portion includes a plurality of holes spaced apart longitudinally, the plurality of holes distal to the marker band and proximal to the distal tip; wherein the distal portion includes an elliptically-shaped hole distal to the marker band; and wherein the marker band and the distal tip are configured to be visible by echogenicity and/or echopacity. The distal portion may include a circular loop in a longitudinal segment of the distal portion such that the distal tip at least approximates an outer surface of the tubular body proximal to the distal tip, the circular loop including an inner circumference and an outer circumference; wherein the inner circumference includes the plurality of holes; and wherein the outer circumference includes an elliptically-shaped hole distal to the marker band. The marker band and the distal tip each may include from about 70 weight % to about 90 weight % of tungsten. The marker band and the distal tip each may include from about 40 weight % to about 60 weight % of bismuth.

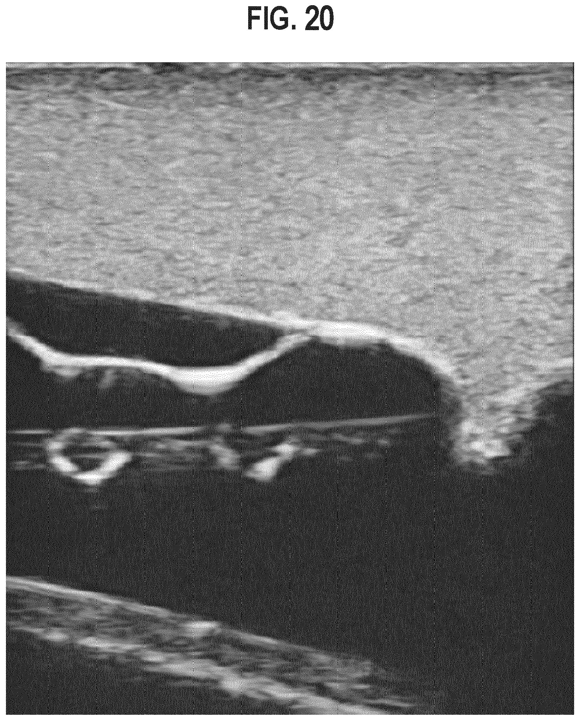

Referring now to FIG. **20****,** an ultrasound of yet another example of a drainage catheter according to the principles of the present disclosure upon insertion into a phantom pleural cavity filled with fluid is illustrated. Upon insertion, some of the plurality of holes of the distal portion are visible under ultrasound in relative contrast. Due to the echogenicity of the example of the drainage catheter shown under ultrasound in FIG. **20****,** the plurality of holes, and the interfaces between the echogenic catheter and the voids created by the plurality of holes, are very clearly visible under ultrasound, which is not demonstrated by currently available drainage catheters. Currently, physicians use movement or fluid flow or tiny air bubbles to visualize the drainage catheter interface or holes in the drainage catheter of the state of the art. The drainage catheter illustrated in FIG. **20** includes primarily barium sulfate as a filler material at a lower concentration than ideal for ultrasound so as to provide a control for effective qualitative comparison to ultrasound images of drainage catheters with higher concentrations of filler material.

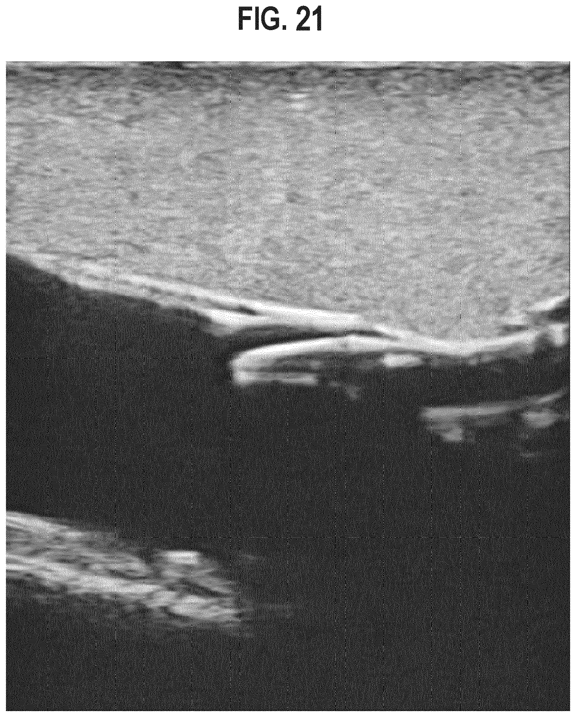

Referring now to FIG. **21****,** an ultrasound of yet another example of a drainage catheter according to the principles of the present disclosure upon insertion into a phantom pleural cavity filled with fluid is illustrated. Upon withdrawal of the drainage catheter from the phantom pleural cavity, the tapered, distal tip of the device is visible in relative contrast under ultrasound. The echogenic material of the example of the drainage catheter shown under ultrasound in FIG. **21** makes the distal tip visible under ultrasound, which is a property not demonstrated by currently available drainage catheters.

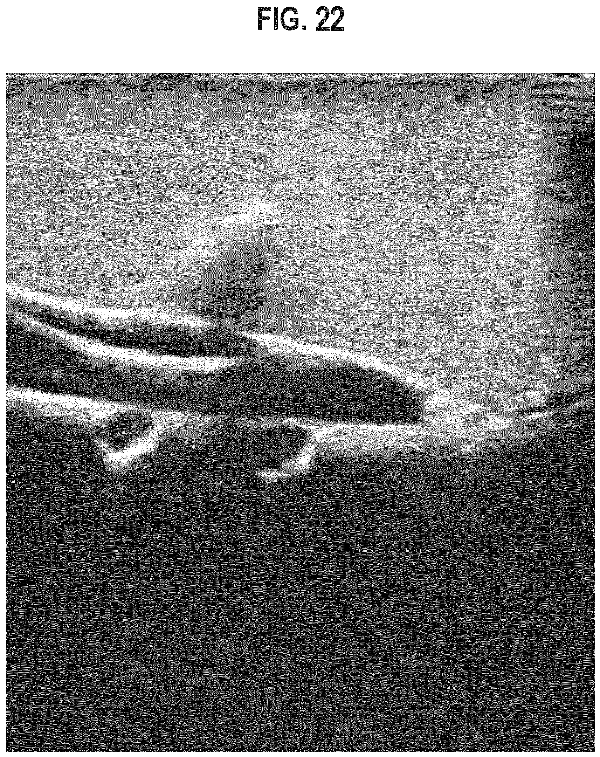

Referring now to FIG. **22****,** an ultrasound of yet another example of a drainage catheter according to the principles of the present disclosure upon insertion into a phantom pleural cavity filled with fluid is illustrated. Some of the plurality of holes of the distal portion are visible under ultrasound in high contrast. The drainage catheter illustrated in FIG. **22** includes primarily hollow glass microspheres as sa filler material to enhance echogenicity as compared to the echnogenicity of the drainage catheter illustrated in FIG. **20****.**

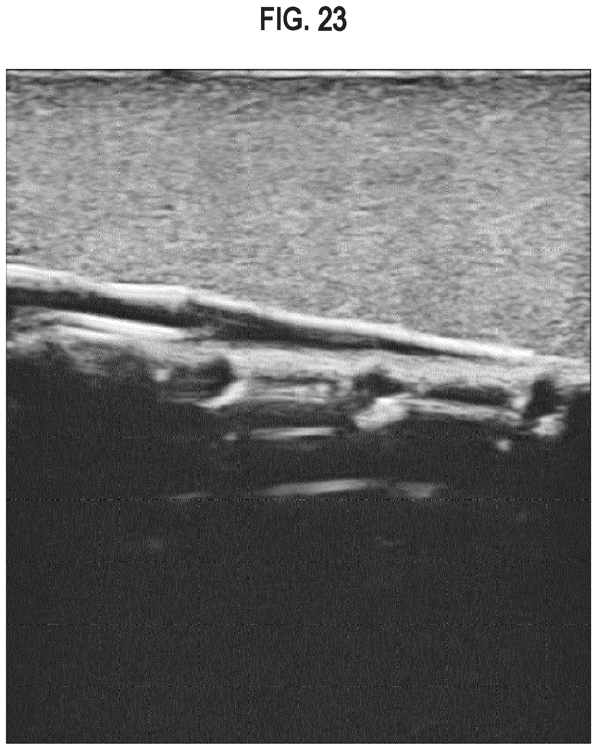

Referring now to FIG. **23****,** an ultrasound of yet another example of a drainage catheter according to the principles of the present disclosure upon insertion into a phantom pleural cavity filled with fluid is illustrated. Some of the plurality of holes of the distal portion are visible under ultrasound in high contrast. The drainage catheter illustrated in FIG. **23** includes an echogenic inner layer with a smooth outer layer of non-echogenic material. The bilayered material of the example of the drainage catheter shown under ultrasound in FIG. **23** provides improved visibility under ultrasound compared to currently available drainage catheters. The echogenic material of the inner layer improves ultrasound visibility. Further, the interface between the echogenic inner layer and the smooth outer layer in the bilayered drainage catheter is very vivid under ultrasound.

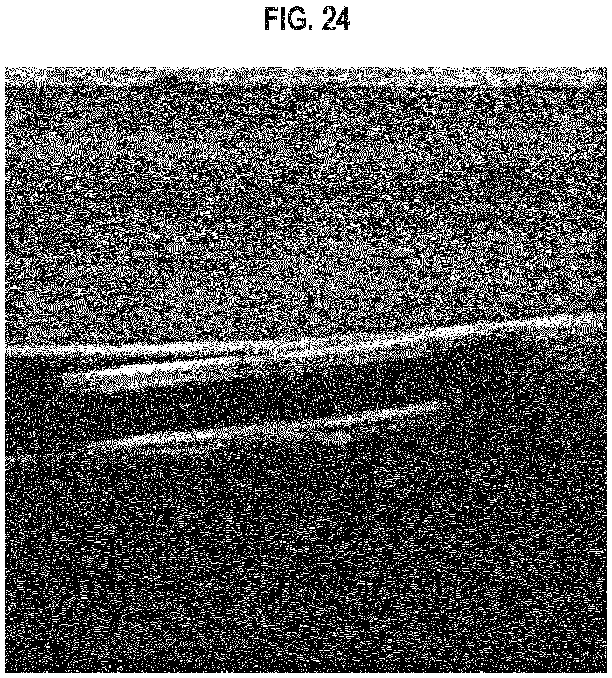

Referring now to FIG. **24****,** an ultrasound of an example of a state-of-the-art catheter tube, for comparison, upon insertion into a phantom pleural cavity filled with fluid is illustrated. The edges of the catheter tube are visible under ultrasound in mild contrast in FIG. **24****.** The catheter tube shown under ultrasound in FIG. **24** includes primarily barium sulfate as a filler material at a lower concentration than ideal for ultrasound so as to provide a control for effective qualitative comparison to ultrasound images of examples of drainage catheters according to the principles of the present disclosure.

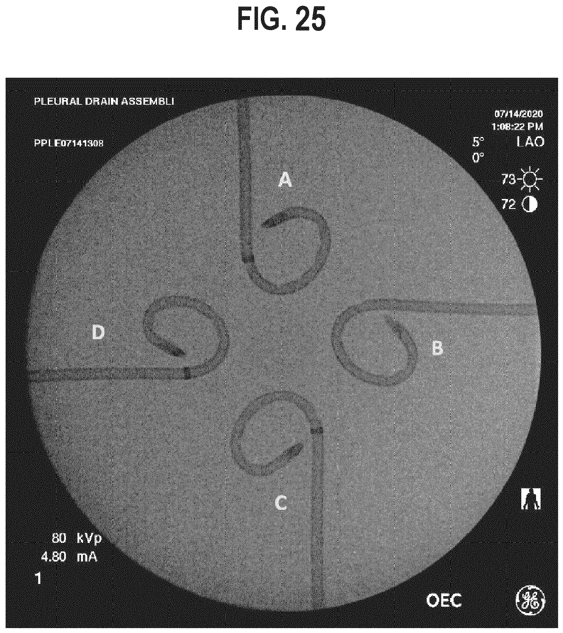

Referring now to FIG. **25****,** examples of drainage catheters of FIGs. **20** - **23** as shown under fluoroscopy are illustrated. Drainage catheters A, C, and D correspond to the drainage catheters illustrated under ultrasound in FIGs. **20****,** **22****,** and **23****,** respectively, and include radiopaque marker bands that are not echogenic. Drainage catheter B corresponds to the drainage catheter illustrated under ultrasound in FIG. **21****,** and includes an echogenic marker band with negligible radiopacity.

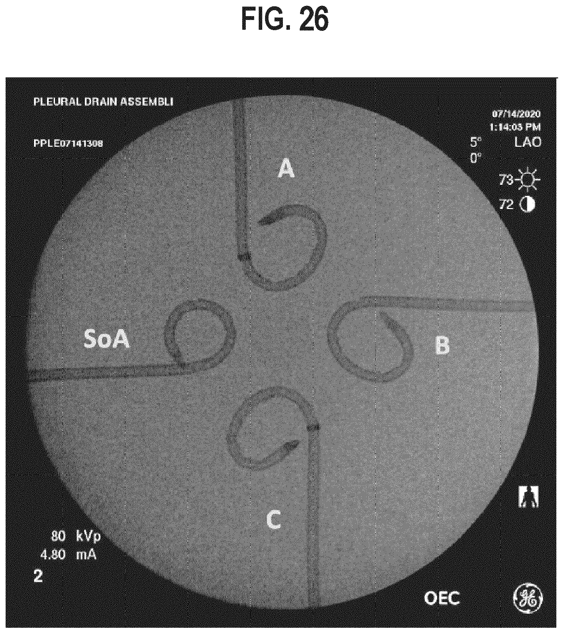

Referring now to FIG. **26****,** examples of drainage catheters of FIGs. **20** - **22** and **24** as shown under fluoroscopy are illustrated. Drainage catheters A and C correspond to the drainage catheters illustrated under ultrasound in FIGs. **20** and **22****,** respectively, and include radiopaque marker bands that are not echogenic. Drainage catheter B corresponds to the drainage catheter illustrated under ultrasound in FIG. **21****,** and includes an echogenic marker band with negligible radiopacity. The drainage catheter labeled "SoA" ("state-of-the-art") corresponds to the drainage catheter illustrated under ultrasound in FIG. **24** and includes neither an echogenic nor a radiopaque marker band. The marker bands of the examples of drainage catheters of FIGs. **20** - **22** are more visible under ultrasound compared to currently available catheters, as shown by the ultrasound of SoA. The relatively larger size of the tungsten particulate additive in the marker bands of the examples of drainage catheters the ultrasounds of which are shown in FIGs. **20** - **22** make the marker bands visible under ultrasound.

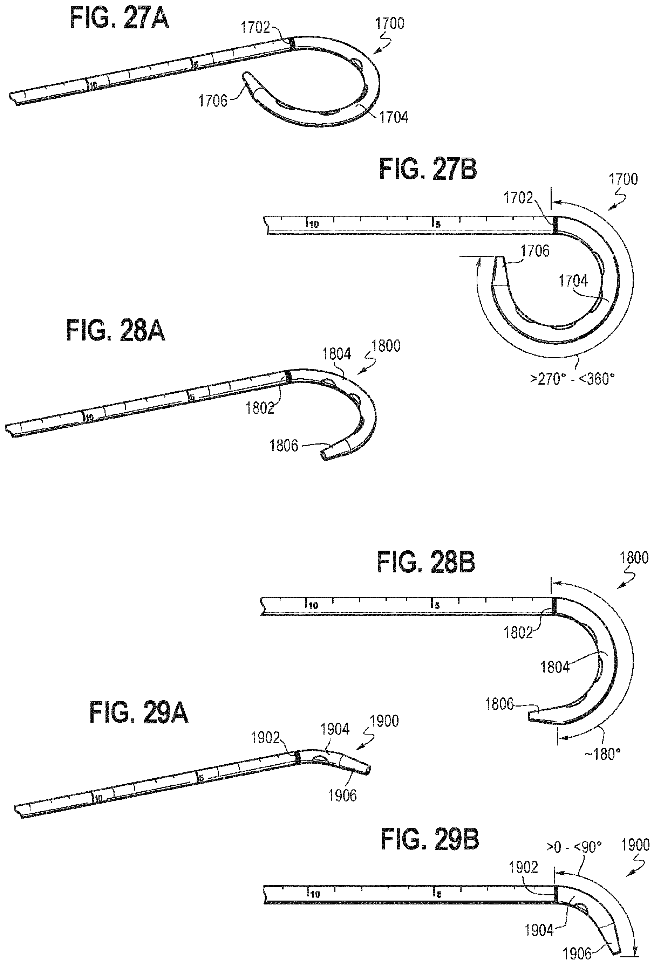

Referring now to FIG. **27A****,** a perspective view of a distal portion **1704** of yet another example of a drainage catheter **1700** is illustrated. FIG. **27B** illustrates a side view of distal portion **1704,** with a degree of curvature between marker band **1702** and distal tip **1706** of greater than 270 degrees and less than 360 degrees. Distal tip **1706** at least approximates the outer surface of drainage catheter **1700.** Accordingly, drainage catheter **1700** may resemble the shape of a "shepherd's hook."

Referring now to FIG. **28A****,** a perspective view of a distal portion **1804** of yet another example of a drainage catheter **1800** is illustrated. FIG. **28B** illustrates a side view of distal portion **1804,** with a degree of curvature between marker band **1802** and distal tip **1806** of approximately 180 degrees. According, drainage catheter **1800** may resemble the shape of a "candy cane."

Referring now to FIG. **29A****,** a perspective view of a distal portion **1904** of yet another example of a drainage catheter **1900** is illustrated. FIG. **29B** illustrates a side view of distal portion **1904,** with a degree of curvature between marker band **1902** and distal tip **1906** of greater than 0 degrees and less than 90 degrees. Accordingly, drainage catheter **1900** may resemble the shape of a "hockey stick."

Although the present disclosure has been described with reference to examples and the accompanying drawings, the present disclosure is not limited thereto, but may be variously modified and altered by those skilled in the art to which the present disclosure pertains without departing from the spirit and scope of the present disclosure.

The subject-matter of the disclosure may also relate, among others, to the following aspects:

A first aspect relates to a drainage catheter, comprising: a tubular body extending along a longitudinal axis, the tubular body comprising: an outer surface; a lumen extending longitudinally throughout the tubular body, the lumen comprising a lumen surface; and a distal portion comprising a tapered distal tip; and wherein the distal portion has a degree of curvature of greater than 0 degrees; wherein the distal portion comprises a plurality of holes proximal to the distal tip and spaced apart longitudinally, and an accessory hole proximal to the plurality of holes; wherein the tubular body is configured to be visible by echogenicity and/or echopacity, or the lumen surface or the outer surface is covered by a layer configured to be visible by echogenicity and/or echopacity.

A second aspect relates to the drainage catheter of aspect **1,** wherein the distal portion comprises a circular loop in a longitudinal segment of the tubular body such that the distal tip at least approximates an outer surface of the tubular body proximal to the distal tip, the circular loop comprising an inner circumference and an outer circumference; wherein the inner circumference comprises the plurality of holes; and wherein the outer circumference com prises the accessory hole.

A third aspect relates to the drainage catheter of any preceding aspect, wherein the distal portion is configured to be visible by echogenicity and/or echopacity, or is covered by the layer, the layer extending longitudinally from the distal tip to proximal to the accessory hole.

A fourth aspect relates to the drainage catheter of any preceding aspect, wherein the tubular body or the layer comprises from about 70 weight % to about 90 weight % of tungsten uniformly distributed in the tubular body or the layer.

A fifth aspect relates to the drainage catheter of any one of aspects **1** to **3,** wherein the tubular body or the layer comprises from about 40 weight % to about 60 weight % of bismuth uniformly distributed in the tubular body or the layer.

A sixth aspect relates to the drainage catheter of any preceding aspect, wherein the tubular body or the layer is configured to provide a visual confirmation to an operator of a depth of the drainage catheter in a pleural cavity of a patient past the accessory hole.

A seventh aspect relates to the drainage catheter of any preceding aspect, wherein the distal portion has a degree of curvature of greater than 360 degrees.

An eighth aspect relates to a drainage catheter, comprising: a tubular body extending along a longitudinal axis, the tubular body comprising: an outer surface; a lumen extending longitudinally throughout the tubular body, the lumen comprising a lumen surface; and a distal portion comprising a tapered distal tip; wherein the distal portion has a degree of curvature of about 0 degrees; wherein the distal portion com prises a plurality of holes proximal to the distal tip and spaced apart longitudinally and circumferentially about a circumference of the distal portion; wherein the tubular body is configured to be visible by echogenicity and/or echopacity, or the lumen surface or the outer surface is covered by a layer configured to be visible by echogenicity and/or echopacity.

A ninth aspect relates to the drainage catheter of aspect **8,** wherein the tubular body or the layer comprises from about 70 weight % to about 90 weight % of tungsten uniformly distributed in the tubular body or the layer.

A tenth aspect relates to the drainage catheter of aspect **8,** wherein the tubular body or the layer comprises from about 40 weight % to about 60 weight % of bismuth uniformly distributed in the tubular body or the layer.

An eleventh aspect relates to the drainage catheter of any one of aspects **8** to **10,** wherein the distal portion is configured to be visible by echogenicity and/or echopacity, or is covered by the layer, the layer extending longitudinally from the distal tip to proximal to the plurality of holes.

A twelfth aspect relates to the drainage catheter of any one of aspects **8** to **11,** wherein the marker band is configured to provide a visual confirmation to an operator of a depth of the drainage catheter in a pleural cavity of a patient past the plurality of holes.

A thirteenth aspect relates to a drainage catheter, comprising: a tubular body extending along a longitudinal axis, the tubular body comprising: three concentric layers of material extending longitudinally, the layers comprising an exterior layer, a middle layer, and an interior layer; a lumen extending longitudinally throughout the tubular body; a tapered distal tip; and a plurality of holes proximal to the distal tip and spaced apart longitudinally; wherein the exterior layer, the middle layer, or the interior layer is a layer configured to be visible by echogenicity and/or echopacity.

A fourteenth aspect relates to the drainage catheter of aspect **13,** wherein the layer configured to be visible by echogenicity and/or echopacity comprises from about 70 weight % to about 90 weight % of tungsten uniformly distributed in the layer.

A fifteenth aspect relates to the drainage catheter of aspect **13,** wherein the layer configured to be visible by echogenicity and/or echopacity comprises from about 40 weight % to about 60 weight % of bismuth uniformly distributed in the layer.

A sixteenth aspect relates to the drainage catheter of any one of aspects **13** to **15,** wherein the layer configured to be visible by echogenicity and/or echopacity provides a visual confirmation to an operator of a depth of the drainage catheter in a pleural cavity of a patient past the plurality of holes.

A seventeenth aspect relates to the drainage catheter of any one of aspects **13** to **16,** wherein the tubular body comprises a distal portion comprising the distal tip and the plurality of holes; and wherein the distal portion has a degree of curvature of greater than 0 degrees.

An eighteenth aspect relates to the drainage catheter of any one of aspects **13** to **17,** wherein the distal portion comprises an elliptically-shaped hole proximal to the plurality of holes.

A nineteenth aspect relates to the drainage catheter of any one of aspects **13** to **18,** wherein the tubular body comprises a distal portion comprising the distal tip and the plurality of holes; and wherein the distal portion comprises the three concentric layers of material.

A twentieth aspect relates to the drainage catheter of any one of aspects **13** to **19,** wherein the tubular body comprises a distal portion comprising the distal tip and the plurality of holes; wherein the distal portion has a degree of curvature of about 0 degrees; and wherein the plurality of holes is spaced apart circumferentially about a circumference of the tubular body.

A twenty-first aspect relates to a drainage catheter, comprising: a tubular body extending along a longitudinal axis, the tubular body comprising: a lumen extending longitudinally throughout the tubular body; a distal portion comprising a tapered distal tip; and a marker band encircling the tubular body proximal to the distal tip; wherein the distal portion has a degree of curvature of greater than 0 degrees; wherein the distal portion comprises a plurality of holes spaced apart longitudinally, the plurality of holes distal to the marker band and proximal to the distal tip; wherein the distal portion comprises an elliptically-shaped hole distal to the marker band; wherein the marker band and the distal tip are configured to be visible by echogenicity and/or echopacity.

A twenty-second aspect relates to the drainage catheter of aspect **21,** wherein the distal portion comprises a circular loop in a longitudinal segment of the tubular body such that the distal tip at least approximates an outer surface of the tubular body proximal to the distal tip, the circular loop comprising an inner circumference and an outer circumference; wherein the inner circumference comprises the plurality of holes; and wherein the outer circumference comprises the elliptically-shaped hole.

A twenty-third aspect relates to the drainage catheter of any of aspects **21** or **22,** wherein the marker band and the distal tip each comprise from about 70 weight % to about 90 weight % of tungsten.

A twenty-fourth aspect relates to the drainage catheter of any of aspects **21** or **22,** wherein the marker band and the distal tip each comprise from about 40 weight % to about 60 weight % of bismuth.

A twenty-fifth aspect relates to the drainage catheter of any of aspects **21** or **22,** wherein the tubular body comprises about 1 weight % to about 10 weight % of a particulate additive comprising spheres, microspheres, and/or nanospheres.

A twenty-sixth aspect relates to the drainage catheter of aspect **25,** wherein the particulate additive comprises glass microspheres of a particulate size of from about 2 microns to about 100 microns.

A twenty-seventh aspect relates to the drainage catheter of any of aspects **21** to **26,** wherein the tubular body comprises graduated depth indicator lines spaced apart longitudinally and proximal to the plurality of holes.

A twenty-eighth aspect relates to the drainage catheter of any of aspects **21** to **27,** wherein the marker band is configured to provide a visual confirmation to an operator of a depth of the drainage catheter in a pleural cavity of a patient past a most proximal hole of the plurality of holes.

A twenty-ninth aspect relates to the drainage catheter of any of aspects **21** to **28,** wherein the marker band is at least 5 millimeters wide longitudinally.

A thirtieth aspect relates to a drainage catheter, comprising: a tubular body extending along a longitudinal axis, the tubular body comprising: a lumen extending longitudinally throughout the tubular body, the lumen having a cross-sectional inner diameter; a distal portion comprising a tapered distal tip; and a marker band encircling the tubular body proximal to the distal tip; wherein the distal portion has a degree of curvature of 0 degrees; wherein the distal portion comprises a plurality of holes spaced apart longitudinally and circumferentially about the circumference of the distal portion, the plurality of holes distal to the marker band and proximal to the distal tip; and wherein the marker band and the distal tip are configured to be visible by echogenicity and/or echopacity.

A thirty-first aspect relates to the drainage catheter of aspect **30,** wherein the marker band and the distal tip each comprises from about 70 weight % to about 90 weight % of tungsten.

A thirty-second aspect relates to the drainage catheter of aspect **30,** wherein the marker band and the distal tip each comprise from about 40 weight % to about 60 weight % of bismuth.

A thirty-third aspect relates to the drainage catheter of aspect **30,** wherein the tubular body comprises from about 1 weight % to about 10 weight % of a particulate additive comprising spheres, microspheres, and/or nanospheres.

A thirty-fourth aspect relates to the drainage catheter of any of aspects **30** to **33,** wherein the particulate additive comprises glass microspheres of a particle size of from about 2 microns to about 100 microns.

A thirty-fifth aspect relates to the drainage catheter of any of aspects **30** to **34,** wherein the tubular body comprises graduated depth indicator lines spaced apart longitudinally and proximal to the plurality of holes.

A thirty-sixth aspect relates to the drainage catheter of any of aspects **30** to **35** wherein the marker band is configured to provide a visual confirmation to an operator of a depth of the drainage catheter in a pleural cavity of a patient past a most proximal hole of the plurality of holes.

A thirty-seventh aspect relates to a drainage catheter, comprising: a tubular body extending along a longitudinal axis, the tubular body comprising: a lumen extending longitudinally throughout the tubular body; a distal portion comprising a tapered distal tip; and a marker band encircling the tubular body proximal to the distal tip; wherein the distal portion has a degree of curvature of at least 360 degrees; wherein the distal portion comprises a plurality of holes spaced apart longitudinally, the plurality of holes distal to the marker band and proximal to the distal tip; wherein the distal portion comprises an elliptically-shaped hole distal to the marker band; and wherein the marker band and the distal tip are configured to be visible by echogenicity and/or echopacity.

A thirty-eighth aspect relates to the drainage catheter of aspect **37,** wherein the distal portion comprises a circular loop in a longitudinal segment of the distal portion such that the distal tip at least approximates an outer surface of the tubular body proximal to the distal tip, the circular loop comprising an inner circumference and an outer circumference; wherein the inner circumference comprises the plurality of holes; and wherein the outer circumference comprises an elliptically-shaped hole distal to the marker band.

A thirty-ninth aspect relates to the drainage catheter of any of aspects **37** or **38,** wherein the marker band and the distal tip each comprise from about 70 weight % to about 90 weight % of tungsten.

A fortieth aspect relates to the drainage catheter of any of aspects **37** or **38,** wherein the marker band and the distal tip each comprise from about 40 weight % to about 60 weight % of bismuth.

In addition to the features mentioned in each of the independent aspects enumerated above, some examples may show, alone or in combination, the optional features mentioned in the dependent aspects and/or disclosed in the description above and shown in the figures.

## Claims

1. A drainage catheter, comprising:
a tubular body extending along a longitudinal axis, the tubular body com prising:
an outer surface;
a lumen extending longitudinally throughout the tubular body, the lumen comprising a lumen surface; and
a distal portion comprising a tapered distal tip; and
wherein the distal portion has a degree of curvature of greater than 0 degrees;
wherein the distal portion comprises a plurality of holes proximal to the distal tip and spaced apart longitudinally, and an accessory hole proximal to the plurality of holes;
wherein the tubular body is configured to be visible by echogenicity and/or echopacity, or the lumen surface or the outer surface is covered by a layer configured to be visible by echogenicity and/or echopacity.

2. The drainage catheter of claim 1, wherein the distal portion comprises a circular loop in a longitudinal segment of the tubular body such that the distal tip at least approximates an outer surface of the tubular body proximal to the distal tip, the circular loop comprising an inner circumference and an outer circumference;
wherein the inner circumference comprises the plurality of holes; and
wherein the outer circumference com prises the accessory hole.

3. The drainage catheter of claim 1 or 2, wherein the distal portion is configured to be visible by echogenicity and/or echopacity, or is covered by the layer, the layer extending longitudinally from the distal tip to proximal to the accessory hole.

4. The drainage catheter of any preceding claim, wherein the tubular body or the layer is configured to provide a visual confirmation to an operator of a depth of the drainage catheter in a pleural cavity of a patient past the accessory hole.

5. The drainage catheter of any preceding claim 1, wherein the distal portion has a degree of curvature of greater than 360 degrees.

6. A drainage catheter, comprising:
a tubular body extending along a longitudinal axis, the tubular body com prising:
an outer surface;
a lumen extending longitudinally throughout the tubular body, the lumen comprising a lumen surface; and
a distal portion comprising a tapered distal tip;
wherein the distal portion has a degree of curvature of about 0 degrees;
wherein the distal portion comprises a plurality of holes proximal to the distal tip and spaced apart longitudinally and circumferentially about a circumference of the distal portion;
wherein the tubular body is configured to be visible by echogenicity and/or echopacity, or the lumen surface or the outer surface is covered by layer configured to be visible by echogenicity and/or echopacity.

7. The drainage catheter of claim 8, wherein the distal portion is configured to be visible by echogenicity and/or echopacity, or is covered by the layer, the layer extending longitudinally from the distal tip to proximal to the plurality of holes.

8. The drainage catheter of claim 6 or 7, wherein the tubular body or the layer is configured to provide a visual confirmation to an operator of a depth of the drainage catheter in a pleural cavity of a patient past the plurality of holes.

9. The drainage catheter of any preceding claim, wherein the tubular body or the layer comprises from about 70 weight % to about 90 weight % of tungsten uniformly distributed in the tubular body or the layer.

10. The drainage catheter of any of claims 1 to 8, wherein the tubular body or the layer comprises from about 40 weight % to about 60 weight % of bismuth uniformly distributed in the tubular body or the layer.

11. A drainage catheter, comprising:
a tubular body extending along a longitudinal axis, the tubular body com prising:
three concentric layers of material extending longitudinally, the layers comprising an exterior layer, a middle layer, and an interior layer;
a lumen extending longitudinally throughout the tubular body;
a tapered distal tip; and
a plurality of holes proximal to the distal tip and spaced apart longitudinally;
wherein the exterior layer, the middle layer, or the interior layer is a layer configured to be visible by echogenicity and/or echopacity.

12. The drainage catheter of claim 11, wherein the layer configured to be visible by echogenicity and/or echopacity comprises one of:
(a) from about 70 weight % to about 90 weight % of tungsten uniformly distributed in the layer; and
(b) from about 40 weight % to about 60 weight % of bismuth uniformly distributed in the layer.

13. The drainage catheter of claim 11 or 12, wherein the layer configured to be visible by echogenicity and/or echopacity provides a visual confirmation to an operator of a depth of the drainage catheter in a pleural cavity of a patient past the plurality of holes.

14. The drainage catheter of claim 11,12 or 13, wherein the tubular body comprises a distal portion comprising the distal tip and the plurality of holes; and
wherein the distal portion has a degree of curvature of greater than 0 degrees for example, wherein the distal portion comprises an elliptically-shaped hole proximal to the plurality of holes.

15. The drainage catheter of claim 11,12 or 13, wherein the tubular body comprises a distal portion comprising the distal tip and the plurality of holes; and
wherein the distal portion at least one of:
(a) comprises the three concentric layers of material; and
(b) has a degree of curvature of about 0 degrees wherein the plurality of holes is spaced apart circumferentially about a circumference of the tubular body.
